# EUROPEAN PATENT SPECIFICATION

(11) **EP 1 896 552 B1**
(45) Date of publication and mention of the grant of the patent: **19.05.2010**
(21) Application number: 06762071.6
(22) Date of filing: 19.06.2006
(51) Int. Cl.: C09K 19/32

(54) **LIQUID CRYSTAL MOLECULES COMPRISING HYDROAZULENE STRUCTURES**
FLÜSSIGKRISTALLMOLEKÜLE ENTHALTEND HYDROAZULENGRUPPEN
MOLÉCULES DE CRISTAUX LIQUIDES COMPRENANT DES STRUCTURES D'HYDROAZULÈNES

(30) Priority: 23.06.2005 EP 05013584
(43) Date of publication of application: 12.03.2008
(73) Proprietor: Tetragon-Chemie AG, 9496 Balzers (LI)
(72) Inventor: POHL, Ludwig,, 64285 Darmstadt (DE); HOPF, Henning,, 38106 Braunschweig, (DE); HUSSAIN, Zakir,, 38106 Braunschweig (DE)
(74) Representative: Deckers, Hellmuth Alexander
(86) International application number: PCT/EP2006/005834
(87) International publication number: WO 2006/136345

(56) References cited:
- EP-A- 1 318 185
- PATENT ABSTRACTS OF JAPAN vol. 014, no. 250 (C-0723), 29 May 1990 (1990-05-29) -& JP 02 069441 A (MITSUBISHI KASEI CORP), 8 March 1990 (1990-03-08)
- ESTDALE S E ET AL: "THE AZULENE RING AS A STRUCTURAL ELEMENT IN LIQUID CRYSTALS" JOURNAL OF MATERIALS CHEMISTRY, THE ROYAL SOCIETY OF CHEMISTRY, CAMBRIDGE, GB, vol. 7, no. 3, March 1997 (1997-03), pages 391-401, XP000693112 ISSN: 0959-9428

## Description

The present invention relates to compounds based on hydroazulene structures, to a method of use of the said compounds based on hydroazulene structures to formulate liquid crystalline media, to liquid crystalline media comprising at least one of the said compounds, to liquid crystalline media comprising at least two liquid crystalline compounds, wherein at least one of the said compounds is a compound based on hydroazulene structures, and to liquid crystal displays, preferably electro-optical displays containing these liquid crystalline media.

In JP 02-069 441 A, a coloured liquid crystalline compound based on 2,6-dihydroxyazulene is described which is made by esterification with an acid chloride.

In EP 1 318 185 A1, reactive mesogenes based on one or more azulene groups are described in claim 1, which optionally comprise further unsaturated organic groups that form a conjugated system with the azulene groups.

Compounds having azulene chromophores have also been known from the article "The azulene ring as a structural element in liquid crystals", J. Mater. Chem. 1997, vol. 7, pages 391 to 401.

The optical properties of liquid crystals and of liquid crystalline media comprising compounds exhibiting liquid crystal character can be modified by an externally applied voltage. Such media are therefore used, in particular, as dielectrics in display devices. Electro-optical devices based on liquid crystals have been well known for several decades. A variety of physical effects of liquid crystalline media can be used for display purposes, such as dynamic scattering, DAP (deformation of aligned phases), VA (vertically aligned phases) and MVA (multidomain vertically aligned phases), ECB (electrically controlled birefringence), TN (twisted nematic) structures, STN (supertwisted nematic) structures, SBE (superbirefringence effect), OMI (optical mode interference) and IPS (in-plane-switching).

The most common display devices are TN or so-called Schadt-Helfrich cells. The interior surfaces of two transparent sheets or plates such as preferably glass are coated with a structured transparent electrode layer, usually indium tin oxide sputtered onto the surface. The structure of the electrode layer corresponds to the display segments. Between these layers, a liquid crystalline mixture is placed, the layer thickness of this mixture being approximately from 3 µm to 10 µm. Polymer layers with a ridged surface structure are placed between each electrode layer and the liquid crystal medium to impart a preferential orientation, the ridges in the two layers being in contact with the liquid crystal material being mutually perpendicular. The alignment director, the vector pointing into the orientation direction, therefore makes a 90° turn ("twisted nematic cell") within the liquid crystal medium. A higher extent of rotation such as 180° or 270° ("supertwisted nematic cell") is also possible and preferred, such cells are inexpensive and offer high resolution, at lower viewing angles and slower switching times. Crossed polariser plates are added to the outside surfaces of the transparent sheets. Due to the rotation of the alignment director, and hence, the polarisation plane of light passing through this layer arrangement, the whole assembly transmits light. As soon as an external voltage is applied to the electrodes, reorientation of the liquid crystals occurs, the director pointing in the direction perpendicular to the planes of the cell. The direction of polarisation is no longer rotated, and the area in the cell between the electrodes appears black.

Liquid crystalline media usually comprise at least one liquid crystalline compound, and are also referred to as liquid crystal materials in the context of the present invention. Liquid crystal materials must generally have good chemical and thermal stability and good stability to electric fields and electromagnetic radiation. Furthermore, the liquid crystal materials should have low viscosity, especially rotational viscosity, and give short response times, low threshold voltages and high contrast in the cells. They should furthermore have a suitable mesophase, for example a nematic mesophase for the abovementioned cells, at conventional operating temperatures, i.e. in the broadest possible range above and below room temperature. Since liquid crystals are generally used as mixtures of a plurality of components, it is important that the components are readily miscible with one another. Further properties, such as the electrical resistivity, the dielectric and the optical anisotropy, must satisfy various requirements depending on the cell type and area of application. Thus, materials for twisted nematic cells must have a positive dielectric anisotropy and a high electrical resistivity.

For example, liquid crystalline media having a large positive dielectric anisotropy, a broad nematic phase temperature range, high electrical resistivity, and good UV and temperature stability are desired for matrix liquid crystal displays containing integrated non-linear elements for switching individual pixels. Matrix liquid crystal (MLC) displays of this type have been known. Non-linear elements which can be used for individual switching of the individual pixels may be passive elements such as varistors or diodes, or active elements such as transistors. This latter case is referred to as an "active-matrix liquid-crystal display (AM-LCD)".

In TFT (thin film transistor) displays, the electro-optical effect utilised is usually the TN effect. A distinction is made between TFTs comprising compound semiconductors, for example CdSe, and TFTs based on polycrystalline or amorphous silicon.

The TFT matrix is applied to the inside of one glass plate of the display, while the other glass plate carries the transparent counter electrode on its inside. Compared with the size of the pixel electrode, the TFT is very small and has virtually no adverse effect on the image. This technology can also be expanded to fully colour-compatible displays in which a mosaic of red, green, and blue filters is arranged in such a way that each filter element is located opposite a switchable pixel. TFT displays usually operate as TN cells with crossed polarisers and are backlit.

MLC displays of this type are used as displays in notebook computers, for televisions (pocket TVs) or in automobile or aircraft construction. Besides problems regarding the viewing angle dependence of the contrast and the response times, difficulties also arise in these MLC displays due to inadequate electrical resistivity of the liquid crystal mixtures. With decreasing resistivity, the contrast of an MLC display drops, and the problem of "image sticking" can occur. Since the resistivity of a liquid crystal mixture generally drops over the life of an MLC display owing to interaction with the interior surfaces of the display, a high (initial) resistivity is very important in order to obtain acceptable service lives. In particular in the case of mixtures having a low threshold voltage, it was hitherto hard to achieve a very high electrical resistivity since liquid crystalline materials having a high positive dielectric anisotropy Δ ε mostly also have increased electrical conductivity. It is also desirable that the resistivity decreases as little as possible with increasing temperature and after heating and/or UV exposure. Short display response times can only be realised if the liquid crystal mixtures have a small rotational viscosity. For low temperature use of the displays, such as outdoor, automotive or aircraft applications, it is required that crystallisation or the transition to smectic phases do not occur even at low temperatures, and that the temperature dependence of the viscosity is as low as possible.

To this end, liquid crystalline media are desired which have the following properties:
- wide nematic phase range, in particular down to low temperatures, and low temperature dependence of the viscosity for use of the displays even at low temperatures;
- high resistance to UV radiation for long life time of the displays;
- high positive dielectric anisotropy Δ ε for a low threshold voltage Vₜₕ;
- low rotational viscosity γ1 for short response times, and
- either a low optical anisotropy A n matching the optical retardation d × Δ n to the first transmission minimum - for which d × A n is 0.5 µm, d being the cell length - of the characteristic of a twisted nematic cell between parallel polarisers as calculated by Gooch and Tarry to achieve a low Δ n twisted nematic cell or viewing angle independent panel (VIP) or a high optical birefringence which is needed for IPS displays detailed infra. Molecules that do not exhibit the combination of properties referred to supra, or only to a smaller extent, are less or not at all preferred.

A particularly attractive form of display cell is the so-called IPS, or "in plane switching" cell. While in a conventional TFT liquid crystal display, electrode pairs are mounted one above the other on separate substrates, and only the lower electrode being switched by a thin film transistor, in an IPS display, both electrodes are mounted parallel to each other on the lower substrate, and therefore in the same plane. In the Off-position, the liquid crystal molecules lie parallel to the glass substrates, and to each other, as in conventional TFT displays. They are also aligned to be parallel with the cell's electrode pair. Unlike the molecules at one end of the chains in the common STN liquid crystal displays, none of the molecules are anchored to the lower substrate in the case of an IPS display. Consequently, when a voltage is applied across an electrode pair, the liquid crystal molecules can rotate freely through 90° to align themselves with the field, while remaining parallel to the substrate planes, and the other molecules above and below them. The distinguishing fact in the IPS cell is therefore that all molecules remain parallel to the substrate planes, while in a standard TFT display, the further away a molecule is from the anchored end of a chain, the more does it attempt to align itself with the field between the two electrodes. This variation in the orientation angle of the molecules at different cell depth restricts the viewing angle of the light leaving the cell. With increasing viewing angles, the optical properties of such displays change, making it anisotropic. In an IPS display, such a variation does not occur, and hence, the viewing angle is greatly increased, and the colour stays the same, although the brightness may decrease with increasing viewing angle.

It is therefore an object of the present invention to provide liquid crystalline media for different LCD-types, e.g., IPS-, ECB-, AM-, TN- or STN-displays, which have high long term electrical resistivity and chemical stability, low viscosity, tailored birefringence and dielectric anisotropy while maintaining the other boundary conditions.

Liquid crystal mixtures having a high optical birefringence Δn are desirable for reducing the layer thickness in IPS displays to as low as from 2 µm to 3 µm. Reducing the layer thickness also reduces the response time, and renders the IPS displays fit to display videos. At the same time, new display filling methods lead to higher UV exposure of the liquid crystal mixtures.

It is therefore also a particular object of the invention to provide liquid crystal mixtures having a high birefringence Δn and improved UV stability for IPS displays.

This object is achieved by a liquid crystalline medium comprising at least one compound of the formula

R¹ᵢ-A¹ᵢ-(Z²ᵢ₋A¹ᵢ)ₘ-(Z³ᵢ-A³ᵢ)ₙ-(Z⁴ᵢ-A⁴ᵢ)ₒ-R²ᵢ (i).

At least one of the divalent groups A¹ᵢ, A²ᵢ, A³ᵢ and A⁴ᵢ is a group derived from at least partially hydrogenated azulene, with the bonds to the 2- and 6-, or the 6- and 2-positions. Any of A¹ᵢ, A²ᵢ, A³ᵢ and A⁴ᵢ can be a -7:5- or a -5:7- type at least partially hydrogenated azulene diradical, as shown in the formulae below. The abbreviation "6,2-(per)hydro azulenylene" shall mean a (per)hydro azulene diradical of the structure 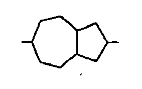 with bonds in the 6- and 2- positions, and the abbreviation "2,6-(per)hydro azulenylene" shall mean a (per)hydro azulene diradical of the structure 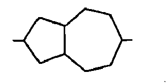 with bonds in the 2- and 6- positions, the mirror image of the structure above.
- R¹ᵢ and R²_{¡}: are each independently selected from the group consisting of hydrogen, an alkyl or alkenyl residue of from 1 to 15 carbon atoms which may optionally be singly substituted with a halogen, -CN or -CF₃ group, wherein in the alkyl or alkenyl residues, one or more methylene -CH₂- groups may be replaced by -O-, -S-, 1,3-cyclobutylene, a keto group -CO-, an ester group -CO-O-, or a carbonate group -O-CO-O-, independently from one another, but in a way that no two oxygen atoms are directly linked to each other,
- R²ᵢ: may also be selected from the group consisting of -CN, -N=C=S, -S-CN, -F, -Cl, -SF₅, -CF₃, -CH₂F, -CHF₂, -O-CF₃, -O-CF₂-CF₂H, and -O-C₂F₅.
- A¹ᵢ, A²ᵢ, A³ᵢ and A⁴ᵢ: are each independently selected from the group consisting of partially or completely hydrogenated 2,6-azulenylene, partially or completely hydrogenated 6,2-azulenylene, trans-1,4-cyclohexylene, 1,4-cyclohexenylene, 1,4-phenylene, 2-fluoro-1,4-phenylene, 3-fluoro-1,4-phenylene, 5-fluoro-1,4-phenylene, 2,3-, 2,5-and 2,6-difluoro-1,4-phenylene, and 3,5-difluoro-1,4-phenylene, 4,4'-diphenylene, 4,4'-biscyclohexylene, tetrahydropyran-2,5- or 3,6-ylene, 1,3- pyrimidine-2,5-diyl, and 1,3-dioxane-2,5-diyl, with the proviso as mentioned above that at least one of the divalent groups A¹ᵢ, A²ᵢ A³ᵢ and A⁴ᵢ is a group derived from at least partially hydrogenated azulene, with the bonds to the 2- and 6-, or 6- and 2-positions.
- Z²ᵢ, Z³ᵢ and Z⁴ᵢ: are independently selected from the group consisting of a single bond, -CH₂-CH₂-,-CF₂-CF₂-, an ester bond -CO-O-, a thioester bond -CO-S-, trans -CH=CH-, -C≡C-, -CH₂-O-and -CF₂-O-, with the proviso that Z^{j}ᵢ may not be an ester or a thioester bond if A^{j-1} ᵢ is a 2,6-(per)hydroazulenylene diradical, or if A^{j}ᵢ is a 6,2-(per)hydroazulenylene diradical
- m, n and o: being 0,1, or 2, independently from each other.

If A⁴ is a cyclohexylene diradical, it is preferred that R² is selected from the group consisting of a C₁- to C₁₅-alkyl, -CN, -CH=CH₂, -F, -O-CH=CF₂, and -CF₃. Likewise, if A⁴ is a phenylene diradical, it is preferred that R² is selected from the group consisting of C₁- to C₁₅-alkyl or -alkoxy radical, -CN, -F, -CF₃ -O-CF₃, -O-CF₂H, -O-CH₂F and -SF₅. Any or both of m and n may assume a value of zero, in this case. Preferred combinations for the latter case where A⁴ is a phenylene diradical, are 3,4-difluoro, 3,4,5-trifluoro, 2,3-difluoro-4-alkyl and 2,3-difluoro-4-alkoxy with a C₁- to C₁₅-alkyl in each case, 3-fluoro-4-trifluoromethyoxy, 3,5-difluoro-4-cyano and 3,5-difluoro 4-difluoromethoxy substituents.

The invention therefore relates to compounds of formula (i), as explained supra.

Preferred compounds of formula (i) are the subject of claims 2 to 4.

A further object of the invention is therefore a method of use of the compounds of formula (i) as a constituent of liquid crystalline media.

Still further objects of the invention are mixtures exhibiting liquid crystal behaviour as claimed in claims 6 to 10 and 12, comprising one or more compounds of claim 1.

The following structures according to formula (i) where at least one fluorine substituent is present in the (per)hydroazulene moiety have been particularly useful as constituents of liquid crystalline media:
2-propyl-6-(3,4,5-trifluorophenyl)-perhydroazulene,
2-propyl-6-(2,3-difluoro-4-ethoxyphenyl)-perhydroazulene,
6-propyl-2-(3,4,5-trifluorophenyl)-perhydroazulene,
6-propyl-2-(2,3-difluoro-4-ethoxyphenyl)-perhydroazulene,
1-fluoro-2-propyl-6-(3,4,5-trifluorophenyl)-perhydroazulene,
1-fluoro-2-propyl-6-(2,3-difluoro-4-ethoxyphenyl)-perhydroazulene,
1-fluoro-6-propyl-2-(3,4,5-trifluorophenyl)-perhydroazulene,
1-fluoro-6-propyl-2-(2,3-difluoro-4-ethoxyphenyl)-perhydroazulene,
4-fluoro-2-propyl-6-(3,4,5-trifluorophenyl)-perhydroazulene,
4-fluoro-2-propyl-6-(2,3-difluoro-4-ethoxyphenyl)-perhydroazulene,
4-fluoro-6-propyl-2-(3,4,5-trifluorophenyl)-perhydroazulene,
4-fluoro-6-propyl-2-(2,3-difluoro-4-ethoxyphenyl)-perhydroazulene,
5-fluoro-2-propyl-6-(3,4,5-trifluorophenyl)-perhydroazulene,
5-fluoro-2-propyl-6-(2,3-difluoro-4-ethoxyphenyl)-perhydroazulene,
5-fluoro-6-propyl-2-(3,4,5-trifluorophenyl)-perhydroazulene,
5-fluoro-6-propyl-2-(2,3-difluoro-4-ethoxyphenyl)-perhydroazulene,
9-fluoro-2-propyl-6-(3,4,5-trifluorophenyl)-perhydroazulene,
9-fluoro-2-propyl-6-(2,3-difluoro-4-ethoxyphenyl)-perhydroazulene,
9-fluoro-6-propyl-2-(3,4,5-trifluorophenyl)-perhydroazulene,
9-fluoro-6-propyl-2-(2,3-difluoro-4-ethoxyphenyl)-perhydroazulene,
1,3-difluoro-2-propyl-6-(3,4,5-trifluorophenyl)-perhydroazulene,
1,3-difluoro-2-propyl-6-(2,3-difluoro-4-ethoxyphenyl)-perhydroazulene,
1,3-difluoro-6-propyl-2-(3,4,5-trifluorophenyl)-perhydroazulene,
1,3-difluoro-6-propyl-2-(2,3-difluoro-4-ethoxyphenyl)-perhydroazulene,
1,4-difluoro-2-propyl-6-(3,4,5-trifluorophenyl)-perhydroazulene,
1,4-difluoro-2-propyl-6-(2,3-difluoro-4-ethoxyphenyl)-perhydroazulene,
1,4-difluoro-6-propyl-2-(3,4,5-trifluorophenyl)-perhydroazulene,
1,4-difluoro-6-propyl-2-(2,3-difluoro-4-ethoxyphenyl)-perhydroazulene,
1,5-difluoro-2-propyl-6-(3,4,5-trifluorophenyl)-perhydroazulene,
1,5-difluoro2Z-propyl-6-(2,3-difluoro-4-ethoxyphenyl)-perhydroazulene,
1,5-difluoro-6-propyl-2-(3,4,5-trifluorophenyl)-perhydroazulene,
1,5-difluoro-6-propyl-2-(2,3-difluoro-4-ethoxyphenyl)-perhydroazulene,
1,8-difluoro-2-propyl-6-(3,4,5-trifluorophenyl)-perhydroazulene,
1,8-difluoro-2-propyl-6-(2,3-difluoro-4-ethoxyphenyl)-perhydroazulene,
1,8-difluoro-6-propyl-2-(3,4,5-trifluorophenyl)-perhydroazulene,
1,8-difluoro-6-propyl-2-(2,3-difluoro-4-ethoxyphenyl)-perhydroazulene,
4,8-difluoro-2-propyl-6-(3,4,5-trifluorophenyl)-perhydroazulene,
4,8-difluoro-2-propyl-6-(2,3-difluoro-4-ethoxyphenyl)-perhydroazulene,
4,8-difluoro-6-propyl-2-(3,4,5-trifluorophenyl)-perhydroazulene,
4,8-difluoro-6-propyl-2-(2,3-difluoro-4-ethoxyphenyl)-perhydroazulene,
4,5-difluoro-2-propyl-6-(3,4,5-trifluorophenyl)-perhydroazulene,
4,5-difluoro-2-propyl-6-(2,3-difluoro-4-ethoxyphenyl)-perhydroazulene,
4,5-difluoro-6-propyl-2-(3,4,5-trifluorophenyl)-perhydroazulene,
4,5-difluoro-6-propyl-2-(2,3-difluoro-4-ethoxyphenyl)-perhydroazulene,
4,7-difluoro-2-propyl-6-(3,4,5-trifluorophenyl)-perhydroazulene,
4,7-difluoro-2-propyl-6-(2,3-difluoro-4-ethoxyphenyl)-perhydroazulene,
4,7-difluoro-6-propyl-2-(3,4,5-trifluorophenyl)-perhydroazulene,
4,7-difluoro-6-propyl-2-(2,3-difluoro-4-ethoxyphenyl)-perhydroazulene,
5,7-difluoro-2-propyl-6-(3,4,5-trifluorophenyl)-perhydroazulene,
5,7-difluoro-2-propyl-6-(2,3-difluoro-4-ethoxyphenyl)-perhydroazulene,
5,7-difluoro-6-propyl-2-(3,4,5-trifluorophenyl)-perhydroazulene,
5,7-difluoro-6-propyl-2-(2,3-difluoro-4-ethoxyphenyl)-perhydroazulene,
9,10-difluoro-2-propyl-6-(3,4,5-trifluorophenyl)-perhydroazulene,
9,10-difluoro-2-propyl-6-(2,3-difluoro-4-ethoxyphenyl)-perhydroazulene,
9,10-difluoro-6-propyl-2-(3,4,5-trifluorophenyl)-perhydroazulene,
9,10-difluoro-6-propyl-2-(2,3-difluoro-4-ethoxyphenyl)-perhydroazulene,
1,9-difluoro-2-propyl-6-(3,4,5-trifluorophenyl)-perhydroazulene,
1,9-difluoro-2-propyl-6-(2,3-difluoro-4-ethoxyphenyl)-perhydroazulene,
1,9-difluoro-6-propyl-2-(3,4,5-trifluorophenyl)-perhydroazulene,
1,9-difluoro-6-propyl-2-(2,3-difluoro-4-ethoxyphenyl)-perhydroazulene,
3,9-difluoro-2-propyl-6-(3,4,5-trifluorophenyl)-perhydroazulene,
3,9-difluoro-2-propyl-6-(2,3-difluoro-4-ethoxyphenyl)-perhydroazulene,
3,9-difluoro-6-propyl-2-(3,4,5-trifluorophenyl)-perhydroazulene,
3,9-difluoro-6-propyl-2-(2,3-difluoro-4-ethoxyphenyl)-perhydroazulene,
4,9-difluoro-2-propyl-6-(3,4,5-trifluorophenyl)-perhydroazulene,
4,9-difluoro-2-propyl-6-(2,3-difluoro-4-ethoxyphenyl)-perhydroazulene,
4,9-difluoro-6-propyl-2-(3,4,5-trifluorophenyl)-perhydroazulene,
4,9-difluoro-6-propyl-2-(2,3-difluoro-4-ethoxyphenyl)-perhydroazulene,
5,9-difluoro-2-propyl-6-(3,4,5-trifluorophenyl)-perhydroazulene,
5,9-difluoro-2-propyl-6-(2,3-difluoro-4-ethoxyphenyl)-perhydroazulene,
5,9-difluoro-6-propyl-2-(3,4,5-trifluorophenyl)-perhydroazulene,
5,9-difluoro-6-propyl-2-(2,3-difluoro-4-ethoxyphenyl)-perhydroazulene,
7,9-difluoro-2-propyl-6-(3,4,5-trifluorophenyl)-perhydroazulene,
7,9-difluoro-2-propyl-6-(2,3-difluoro-4-ethoxyphenyl)-perhydroazulene,
7,9-difluoro-6-propyl-2-(3,4,5-trifluorophenyl)-perhydroazulene,
7,9-difluoro-6-propyl-2-(2,3-difluoro-4-ethoxyphenyl)-perhydroazulene,
8,9-difluoro-2-propyl-6-(3,4,5-trifluorophenyl)-perhydroazulene,
8,9-difluoro-2-propyl-6-(2,3-difluoro-4-ethoxyphenyl)-perhydroazulene,
8,9-difluoro-6-propyl-2-(3,4,5-trifluorophenyl)-perhydroazulene,
8,9-difluoro-6-propyl-2-(2,3-difluoro-4-ethoxyphenyl)-perhydroazulene,
1,4,7-trifluoro-2-propyl-6-(3,4,5-trifluorophenyl)-perhydroazulene,
1,4,7-trifluoro-2-propyl-6-(2,3-difluoro-4-ethoxyphenyl)-perhydroazulene,
1,4,7-trifluoro-6-propyl-2-(3,4,5-trifluorophenyl)-perhydroazulene,
1,4,7-trifluoro-6-propyl-2-(2,3-difluoro-4-ethoxyphenyl)-perhydroazulene,
3,4,7-trifluoro-2-propyl-6-(3,4,5-trifluorophenyl)-perhydroazulene,
3,4,7-trifluoro-2-propyl-6-(2,3-difluoro-4-ethoxyphenyl)-perhydroazulene,
3,4,7-trifluoro-6-propyl-2-(3,4,5-trifluorophenyl)-perhydroazulene,
3,4,7-trifluoro-6-propyl-2-(2,3-difluoro-4-ethoxyphenyl)-perhydroazulene,
1,3,4-trifluoro-2-propyl-6-(3,4,5-trifluorophenyl)-perhydroazulene,
1,3,4-trifluoro-2-propyl-6-(2,3-difluoro-4-ethoxyphenyl)-perhydroazulene,
1,3,4-trifluoro-6-propyl-2-(3,4,5-trifluorophenyl)-perhydroazuiene,
1,3,4-trifluoro-6-propyl-2-(2,3-difluoro-4-ethoxyphenyl)-perhydroazulene,
1,3,5-trifluoro-2-propyl-6-(3,4,5-trifluorophenyl)-perhydroazulene,
1,3,5-trifluoro-2-propyl-6-(2,3-difluoro-4-ethoxyphenyl)-perhydroazulene,
1,3,5-trifluoro-6-propyl-2-(3,4,5-trifluorophenyl)-perhydroazulene,
1,3,5-trifluoro-6-propyl-2-(2,3-difluoro-4-ethoxyphenyl)-perhydroazulene,
1,4,5-trifluoro-2-propyl-6-(3,4,5-trifluorophenyl)-perhydroazulene,
1,4,5-trifluoro-2-propyl-6-(2,3-difluoro-4-ethoxyphenyl)-perhydroazulene,
1,4,5-trifluoro-6-propyl-2-(3,4,5-trifluorophenyl)-perhydroazulene,
1,4,5-trifluoro-6-propyl-2-(2,3-difluoro-4-ethoxyphenyl)-perhydroazulene,
1,4,7-trifluoro-2-propyl-6-(3,4,5-trifluorophenyl)-perhydroazulene,
1,4,7-trifluoro-2-propyl-6-(2,3-difluoro-4-ethoxyphenyl)-perhydroazulene,
1,4,7-trifluoro-6-propyl-2-(3,4,5-trifluorophenyl)-perhydroazulene,
1,4,7-trifluoro-6-propyl-2-(2,3-difluoro-4-ethoxyphenyl)-perhydroazulene,
1,4,8-trifluoro-2-propyl-6-(3,4,5-trifluorophenyl)-perhydroazulene,
1,4,8-trifluoro-2-propyl-6-(2,3-difluoro-4-ethoxyphenyl)-perhydroazulene,
1,4,8-trifluoro-6-propyl-2-(3,4,5-trifluorophenyl)-perhydroazulene,
1,4,8-trifluoro-6-propyl-2-(2,3-difluoro-4-ethoxyphenyl)-perhydroazulene,
1,5,7-trifluoro-2-propyl-6-(3,4,5-trifluorophenyl)-perhydroazulene,
1,5,7-trifluoro-2-propyl-6-(2,3-difluoro-4-ethoxyphenyl)-perhydroazulene,
1,5,7-trifluoro-6-propyl-2-(3,4,5-trifluorophenyl)-perhydroazulene,
1,5,7-trifluoro-6-propyl-2-(2,3-difluoro-4-ethoxyphenyl)-perhydroazulene,
1,3,9-trifluoro-2-propyl-6-(2,3-difluoro-4-ethoxyphenyl)-perhydroazulene,
1,3,9-trifluoro-6-propyl-2-(3,4,5-trifluorophenyl)-perhydroazulene,
1,3,9-trifluoro-6-propyl-2-(2,3-difluoro-4-ethoxyphenyl)-perhydroazulene,
1,3,9-trifluoro-6-propyl-2-(2,3-difluoro-4-ethoxyphenyl)-perhydroazulene,
1,4,9-trifluoro-2-propyl-6-(3,4,5-trifluorophenyl)-perhydroazulene,
1,4,9-trifluoro-2-propyl-6-(2,3-difluoro-4-ethoxyphenyl)-perhydroazulene,
1,4,9-trifluoro-6-propyl-2-(3,4,5-trifluorophenyl)-perhydroazulene,
1,4,9-trifluoro-6-propyl-2-(2,3-difluoro-4-ethoxyphenyl)-perhydroazulene,
1,5,9-trifluoro-2-propyl-6-(3,4,5-trifluorophenyl)-perhydroazulene,
1,5,9-trifluoro-2-propyl-6-(2,3-difluoro-4-ethoxyphenyl)-perhydroazulene,
1,5,9-trifluoro-6-propyl-2-(3,4,5-trifluorophenyl)-perhydroazulene,
1,5,9-trifluoro-6-propyl-2-(2,3-difluoro-4-ethoxyphenyl)-perhydroazulene,
1,7,9-trifluoro-2-propyl-6-(3,4,5-trifluorophenyl)-perhydroazulene,
1,7,9-trifluoro-2-propyl-6-(2,3-difluoro-4-ethoxyphenyl)-perhydroazulene,
1,7,9-trifluoro-6-propyl-2-(3,4,5-trifluorophenyl)-perhydroazulene,
1,7,9-trifluoro-6-propyl-2-(2,3-difluoro-4-ethoxyphenyl)-perhydroazulene,
1,8,9-trifluoro-2-propyl-6-(3,4,5-trifluorophenyl)-perhydroazulene,
1,8,9-trifluoro-2-propyl-6-(2,3-difluoro-4-ethoxyphenyl)-perhydroazulene,
1,8,9-trifluoro-6-propyl-2-(3,4,5-trifluorophenyl)-perhydroazulene,
1,8,9-trifluoro-6-propyl-2-(2,3-difluoro-4-ethoxyphenyl)-perhydroazulene,
1,9,10-trifluoro-2-propyl-6-(3,4,5-trifluorophenyl)-perhydroazulene,
1,9,10-trifluoro-2-propyl-6-(2,3-difluoro-4-ethoxyphenyl)-perhydroazulene,
1,9,10-trifluoro-6-propyl-2-(3,4,5-trifluorophenyl)-perhydroazulene,
1,9,10-trifluoro-6-propyl-2-(2,3-difluoro-4-ethoxyphenyl)-perhydroazulene,
4,9,10-trifluoro-2-propyl-6-(3,4,5-trifluorophenyl)-perhydroazulene,
4,9,10-trifluoro-2-propyl-6-(2,3-difluoro-4-ethoxyphenyl)-perhydroazulene,
4,9,10-trifluoro-6-propyl-2-(3,4,5-trifluorophenyl)-perhydroazulene,
4,9,10-trifluoro-6-propyl-2-(2,3-difluoro-4-ethoxyphenyl)-perhydroazulene,
5,9,10-trifluoro-2-propyl-6-(3,4,5-trifluorophenyl)-perhydroazulene,
5,9,10-trifluoro-2-propyl-6-(2,3-difluoro-4-ethoxyphenyl)-perhydroazulene,
5,9,10-trifluoro-6-propyl-2-(3,4,5-trifluorophenyl)-perhydroazulene,
5,9,10-trifluoro-6-propyl-2-(2,3-difluoro-4-ethoxyphenyl)-perhydroazulene,
1,3,4,8-tetrafluoro-2-propyl-6-(3,4,5-trifluorophenyl)-perhydroazulene,
1,3,4,8-tetrafluoro-2-propyl-6-(2,3-difluoro-4-ethoxyphenyl)-perhydroazulene,
1,3,4,8-tetrafluoro-6-propyl-2-(3,4,5-trifluorophenyl)-perhydroazulene,
1,3,4,8-tetrafluoro-6-propyl-2-(2,3-difluoro-4-ethoxyphenyl)-perhydroazulene,
1,3,5,7-tetrafluoro-2-propyl-6-(3,4,5-trifluorophenyl)-perhydroazulene,
1,3,5,7-tetrafluoro-2-propyl-6-(2,3-difluoro-4-ethoxyphenyl)-perhydroazulene,
1,3,5,7-tetrafluoro-6-propyl-2-(3,4,5-trifluorophenyl)-perhydroazulene,
1,3,5,7-tetrafluoro-6-propyl-2-(2,3-difluoro-4-ethoxyphenyl)-perhydroazulene,
1,7,8,9-tetrafluoro-2-propyl-6-(3,4,5-trifluorophenyl)-perhydroazulene,
1,7,8,9-tetrafluoro-2-propyl-6-(2,3-difluoro-4-ethoxyphenyl)-perhydroazulene,
1,7,8,9-tetrafluoro-6-propyl-2-(3,4,5-trifluorophenyl)-perhydroazulene,
1,7,8,9-tetrafluoro-6-propyl-2-(2,3-difluoro-4-ethoxyphenyl)-perhydroazulene,

The compounds of formula (i) can be used, alone, in mixture with other compounds of formula (i), or in combination with other liquid crystalline materials, to formulate mixtures exhibiting liquid crystal behaviour. As is known in the art, such mixtures generally exhibit broader temperature ranges in which liquid crystal behaviour is displayed. Mixtures may also provide liquid crystal media having lower viscosity, and dielectric properties and electro-optic properties that can be varied by varying the composition.

Still a further object of the invention are liquid crystalline media which preferably comprise a mass fraction of from 1 % to 100 %, particularly preferably from 2 % to 95 %, and especially preferably from 3 % to 85 %, of one or more compounds of formula (i).

Preferred liquid crystalline media comprise a mass fraction of
a) from 1 % to 100 % of one or more compounds of the formula (i),
b) from 0 % to 99 % of one or more compounds which form liquid crystalline phases, of the general formula (ii)

   R¹ᵢᵢ-A¹ᵢᵢ-Z¹ᵢᵢ-(A²ᵢᵢ-Z²ᵢᵢ)ₙ-A³ᵢᵢ-R²ᵢᵢ (ii)

   wherein
   - A¹ᵢᵢ, A²ᵢᵢ and A³ᵢᵢ: are each, independently of one another, selected from the group consisting of trans-1,4-cyclohexylene, 1,4-cyclohexenylene, 1,4-phenylene, 2-fluoro-1,4-phenylene, 3-fluoro-1,4-phenylene, 5-fluoro-1,4-phenylene, 2,3-, 2,5- and 2,6-difluoro-1,4-phenylene, and 3,5-difluoro-1,4-phenylene, 4,4'-diphenylene, 4,4'-biscyclohexylene, 1,3- pyrimidine-2,5-diyl, and 1,3-dioxane-2,5-diyl,
   - -2¹ᵢᵢ- and -Z²ᵢᵢ: represent linking groups selected independently from the group consisting of a single bond, -CH₂-CH₂-, -CF₂-CF₂-, an ester bond -CO-O-, a thioester bond -CO-S-, trans -CH=CH-, -C≡C-, -CH₂-O-and -CF₂-O-,
   - R¹ᵢᵢ and R²ᵢᵢ: are each independently selected from the group consisting of hydrogen, an alkyl residue of from 1 to 15 carbon atoms which may optionally be singly substituted with a halogen, -CN or -CF₃ group, wherein in the alkyl or alkenyl residues, one or more methylene -CH₂- groups may be replaced by -O-, -S-, 1,3-cyclobutylene, a keto group -CO-, an ester group -CO-O-, or a carbonate group -O-CO-O-, independently from one another, but in a way that no two oxygen atoms are directly linked to each other,
   - R²_{¡¡}: may also be selected from the group consisting of -CN, -N=C=S, -S-CN, -F, -Cl, -SF₅,-CF₃, -CH₂F, -CHF₂, -O-CF₃, -O-CF₂-CF₂H, and -O-C₂F₅,
and
c) from 0 % to 15 % of one or more further liquid crystalline compounds.

In a particularly preferred embodiment of the invention, the liquid crystalline medium comprises mass fractions of
a) from 5 % to 50 % of compounds of the formula (i),
b) from 50 % to 95 % of compounds of the general formula (ii)
c) from 0 % to 45 % of further liquid crystalline compounds.

A further object of the invention are liquid crystal display cells comprising a liquid crystal mixture or medium, which medium comprises at least one compound of formula (i).

The compounds of the formula (i) are preferably selected from 2,6-disubstituted hydroazulenes, with a substituent having a +1 effect such as alkyl being in either the 2 or the 6 position of the hydroazulene, and a phenyl or phenylcyclohexyl or a diphenylcyclohexyl system being substituted with at least one group exhibiting a -I effect in the 6 or the 2 position of the hydroazulene.

Preferred are substances according to the formula (i) where
- R¹ᵢ: is an alkyl residue or an alkoxy residue of from 1 to 15 carbon atoms,
- R²ᵢ: is selected from the group consisting of -CN, -N=C=S, -F, -Cl, -CF₃, -CHF₂, CH₂F, -O-CHF₂, -O-CH₂F, -O-CF₂-CF₂H, and -CH=CH₂, the substituent R²ᵢ in each case being cycloaliphatically or aromatically bound, and additionally, from the group consisting of -SF₅, -O-CF₃, and -O- C₂F₅, if R²ᵢ is aromatically bound,
- A¹ᵢ: is dihydro, tetrahydro, hexahydro, octahydro or decahydro azulenylene, preferably tetrahydro, hexahydro, octahydro or decahydro azulenylene,
- A²_{¡}: is 1,4-cyclohexylene or 1,4-phenylene, or if at least one of n and o does not equal zero, 1,3-dioxan-2,5-ylene, tetrahydropyran-2,5- or 3,6-ylene or pyrimidine-2,5-ylene,
- A³ᵢ: 1,4-phenylene, 3-fluoro-1,4-phenylene or 3,5-difluoro-1,4-phenylene,
- A⁴ᵢ: 1,4-phenylene, 3-fluoro-1,4-phenylene, 2,3-difluoro-1,4-phenylene or 3,5-difluoro-1,4-phenylene, and
- Z²ᵢ to Z⁴ᵢ: being independently selected from the group consisting of a single bond, an ester group -CO-O-, an ethylene group -CH₂-CH₂-, ethenylene group -CH=CH-, and an acetylene group -C≡C-,
with the option that if A⁴ᵢ may be chosen to be a hydroazulenylene diradical, then R¹ᵢ and R²ᵢ have to be interchanged, and the meaning of the abbreviations A²ᵢ to A⁴ᵢ to be interchanged accordingly to A³ᵢ to A¹ᵢ.

The invention also relates to electro-optical displays which contain the liquid crystalline media according to the invention, and to the use of these media for electro-optical displays.

The liquid crystal mixtures according to the invention have broader useful ranges of parameters, such as the temperature range, the viewing angles, the values for rotational viscosity, etc., than has been available with the mixtures of the prior art.

Thus, combinations of the mesophase temperature range, rotational viscosity, optical and dielectric anisotropy, elastic constants, and UV stability have been found broader than those of the current prior art materials.

The liquid crystal mixtures which can be used in accordance with the invention are prepared in a manner conventional per se. The dielectrics may also comprise further additives known to the person skilled in the art and described in the literature. For example, pleochroitic dyes and/or chiral dopants can be added. While compounds having carboxylic ester bonds or thiocarboxylic ester bonds in the 6 position of the (per)hydroazulene moiety show unsatisfactory LC behaviour with a very narrow liquid crystalline phase range as pure compounds, it has been found, surprisingly, that mixtures of at least two such compounds with the said ester bonds, but having different structure otherwise, show a broad liquid crystalline temperature range. For this reason, the proviso that an ester or thioester group shall not be directly linked to the 6 position in a perhydroazulene moiety does not apply for mixtures of two or more different liquid crystalline compounds, at least one of them comprising a (per)hydroazulene derived structure.

During the investigations which gave rise to the invention, the following compounds were found to exhibit particularly promising properties when incorporated, as diradicals, into a molecule of the structure (i):
Z-alkyl-6-(4-alkylphenyl)-perhydroazulene
6-alkyl-2-(4-alkylphenyl)-perhydroazulene
2-alkyl-6-(4-alkoxyphenyl)-perhydroazulene
6-alkyl-2-(4-alkoxyphenyl)-perhydroazulene
2-alkyl-6-(2,3-difluoro-4-alkoxyphenyl)-perhydroazulene
6-alkyl-2-(2,3-difluoro-4-alkoxyphenyl)-perhydroazulene
2-alkyl-6-(4-(2,3-difluoro-4-alkoxyphenyl)-cyclohexyl)-perhydroazulene
6-alkyl-2-(4-(2,3-difluoro-4-alkoxyphenyl)-cyclohexyl)-perhydroazulene
2-alkyl-6-(4-cyanophenyl)-perhydroazulene
6-alkyl-2-(4-cyanophenyl)-perhydroazulene
2-alkyl-6-(3,5-difluoro-4-cyanophenyl)-perhydroazulene
6-alkyl-2-(3,5-difluoro-4-cyanophenyl)-perhydroazulene
2-alkyl-6-(4-(4-cyanophenyl)-phenyl)-perhydroazulene
6-alkyl-2-(4-(4-cyanophenyl)-phenyl)-perhydroazulene
2-alkyl-6-(4-fluorophenyl)-perhydroazulene
6-alkyl-2-(4-fluorophenyl)-perhydroazulene
2-alkyl-6-(4-(4-fluorophenyl)-phenyl)-perhydroazulene
6-alkyl-2-(4-(4-fluorophenyl)-phenyl)-perhydroazulene
2-alkyl-6-(3,4-difluorophenyl)-perhydroazulene
6-alkyl-2-(3,4-difluorophenyl)-perhydroazulene
2-alkyl-6-(4-(3,4-difluorophenyl)-phenyl)-perhydroazulene
6-alkyl-2-(4-(3,4-difluorophenyl)-phenyl)-perhydroazulene
2-alkyl-6-(4-(3,4-fluorophenyl)-3,5-difluorophenyl)-perhydroazulene
6-alkyl-2-(4-(3,4-fluorophenyl)-3,5-difluorophenyl)-perhydroazulene
2-alkyl-6-(4-(3,4-difluorophenyl)-cyclohexyl)-perhydroazulene
6-alkyl-2-(4-(3,4-difluorophenyl)-cyclohexyl)-perhydroazulene
2-alkyl-6-(4-(4-(3,4-difluorophenyl)-phenyl)-cyclohexyl)-perhydroazulene
6-alkyl-2-(4-(4-(3,4-difluorophenyl)-phenyl)-cyclohexyl)-perhydroazulene
2-alkyl-6-(3,4,5-trifluorophenyl)-perhydroazulene
6-alkyl-2-(3,4,5-trifluorophenyl)-perhydroazulene
2-alkyl-6-(4-(3,4,5-trifluorophenyl)-phenyl)-perhydroazulene
6-alkyl-2-(4-(3,4,5-trifluorophenyl)-phenyl)-perhydroazulene
2-alkyl-6-(4-(3,4,5-trifluorophenyl)-3-fluorophenyl)-perhydroazulene
6-alkyl-2-(4-(3,4,5-trifluorophenyl)-3-fluorophenyl)-perhydroazulene
2-alkyl-6-(4-(3,4,5-trifluorophenyl)-cyclohexyl)-perhydroazulene
6-alkyl-2-(4-(3,4,5-trifluorophenyl)-cyclohexyl)-perhydroazulene
2-alkyl-6-(4-(4-(3,4,5-trifluorophenyl)-3-fluorophenyl)-cyclohexyl)-perhydroazulene
6-alkyl-2-(4-(4-(3,4,5-trifluorophenyl)-3-fluorophenyl)-cyclohexyl)-perhydroazulene
2-alkyl-6-(4-trifluormethoxyphenyl)-perhydroazulene
6-alkyl-2-(4-trifluormethoxyphenyl)-perhydroazulene
2-alkyl-6-(4-trifluormethoxyphenyl)-perhydroazulene
6-alkyl-2-(4-trifluormethoxyphenyl)-perhydroazulene
2-alkyl-6-(4-(4-trifluormethoxyphenyl)-phenyl)-perhydroazulene
6-alkyl-2-(4-(4-trifluormethoxyphenyl)-phenyl)-perhydroazulene
2-alkyl-6-(4-(4-trifluormethoxyphenyl)-cyclohexyl)-perhydroazulene
6-alkyl-2-(4-(4-trifluormethoxyphenyl)-cyclohexyl)-perhydroazulene
2-alkyl-6-(4-(4-(3,4-difluorophenyl)-phenyl)-cyclohexyl)-perhydroazulene
6-alkyl-2-(4-(4-(3,4-difluorophenyl)-phenyl)-cyclohexyl)-perhydroazulene
2-alkyl-6-(3-fluor-4-trifluormethoxyphenyl)-perhydroazulene
6-alkyl-2-(3-fluor-4-trifluormethoxyphenyl)-perhydroazulene
2-alkyl-6-(3-fluor-4-trifluormethoxyphenyl)-cyclohexyl)-perhydroazulene
6-alkyl-2-(3-fluor-4-trifluormethoxyphenyl)-cyclohexyl)-perhydroazulene
2-alkyl-6-(3,5-difluor-4-difluormethoxyphenyl)-perhydroazulene
6-alkyl-2-(3,5-difluor-4-difluormethoxyphenyl)-perhydroazulene
2-alkyl-6-(3,5-difluor-4-trifluormethoxyphenyl)-cyclohexyl)-perhydroazulene
6-alkyl-2-(3,5-difluor-4-trifluormethoxyphenyl)-cyclohexyl)-perhydroazulene
2-alkyl-6-(4-pentafluorsulfanylphenyl)-perhydroazulene
6-alkyl-2-(4-pentafluorsulfanylphenyl)-perhydroazulene
2-alkyl-6-(4-alkylcyclohexyl)-perhydroazulene
6-alkyl-2-(4-alkylcyclohexyl)-perhydroazulene
2-alkyl-6-(4-trifluormethylcyclohexyl)-perhydroazulene
6-alkyl-2-(4-trifluormethylcyclohexyl)-perhydroazulene

as well as those molecules where, if at least two consecutive rings are bound to the 6 or 2 position of the perhydroazulene derived moiety, either the phenylene or the 1,4-cyclohexylene ring or both are replaced by a 3,5-dioxan-1,4-ylene or a pyrimidine-2,5-ylene diradical,
and all of those molecules mentioned supra where one or more of the direct single bonds Z have been replaced by any of those linking groups selected from the group consisting of -CH₂-CH₂-, -CH₂O-, - CF₂O-, and -CO-O-, with the proviso that an ester group -CO-O- is not directly linked to the 6-position of a (per)hydroazulenylene diradical.

The alkyl residues mentioned supra in the 2 or 6 positions of the (per)hydroazulene derived moiety and those linked to the phenylene or cyclohexylene ring either in the form of alkyl or alkoxy may be different or the same and are independently selected from the group consisting of alkyl radicals of from 1 to 10, preferably from 2 to 9, and particularly preferred from 3 to 8 carbon atoms.

Particularly useful compounds are those that carry fluorine substituents in the (per)hydroazulene moiety, in at least one of the 1, 3, 4, 5, 7, and 8 positions, and especially preferred fluorine substituents in the 9 and 10 positions. Depending on the conformation of the (per)hydroazulene molecule, a high local dipole moment (unsymmetrical conformation with regard to the fluorine atoms, such as on the same side of the plane) or a quadrupole moment (symmetrical conformation with regard to the fluorine atoms, such as on different sides of the plane) are generated which show unusual electro-optic behaviour.

The procedure as explained in Example 1 is a general procedure to make differently substituted perhydroazulenes. Other synthetical routes which have been employed in making the molecules according to the general formulae supra are the following: starting from carboxyl- or carboxylic ester-substituted (per)hydroazulene molecules (cf. molecules 1 and 4 infra), decarboxylation using the HUNSDIECKER reaction or similar reactions to form the bromides or chlorides, and subsequent coupling with other building blocks A as mentioned supra, via a FRIEDEL-CRAFTS or similar reaction, or via a WITTIG reaction where the chloride is converted to the phosphonium salt, and then to the ylide or phosphorane which reacts with aldehyde or ketone intermediates to form an alkene bond between A moieties as mentioned supra, of the type A-CH=CH-A', or via a WITTING-HORNER reaction using triethyl phosphite instead of phosphine. Another versatile synthetical route employed successfully in connection with this invention is converting the halogenides obtained by the HUNSDIECKER reaction, to GRIGNARD reagents which bind to other A moieties. Reduction of the esters, acid chloride or carboxylic acid groups (see infra structures 1 and 4) to the corresponding aldehydes or ketones provides intermediates that are useful in the WITTIG and WITTIG-HORNER reactions mentioned previously to provide new carbon-carbon bonds.

All quantities with the unit "%" are mass fractions (the mass *m*_{B} of a substance B considered, divided by the total mass *m* of the mixture). When a yield is stated, this is the ratio of the mass of the substance obtained, and the mass theoretically expected for a stoichiometric conversion.

The invention is further illustrated by the examples.

### Example 1 Synthesis of 2-alkyl-6-(4-cyanophenyl)-perhydroazulene (= 4-(2-Alkyl-decahydroazulen-6-yl)-benzonitrile) (5)

The following scheme shows the outlined synthetic strategy. A general procedure for the synthesis of compounds of formulae 2 to 5 with various alkyl side chains is also presented.

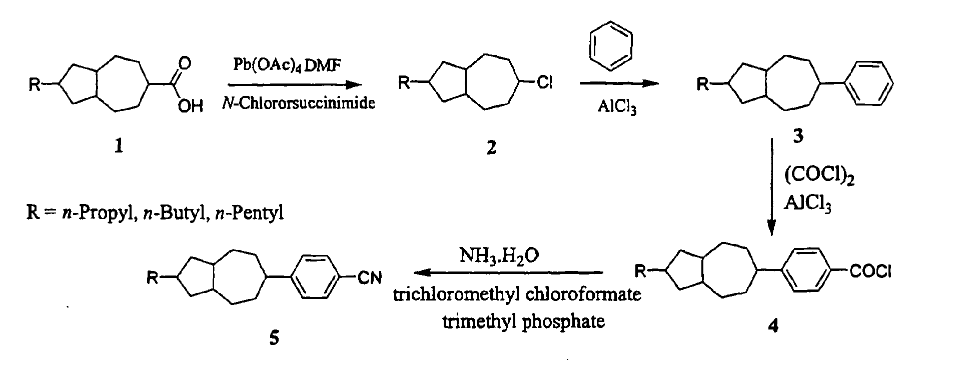

### Example 1.1 6-Chloro-2-propyl-decahydro-azulene (2)

N-Chlorosuccinimide (6.64 g) and acid 1 (8.92 mmol) were dissolved in dimethylformamide (20 ml) and glacial acetic acid (4 ml). The solution was freed of oxygen by repeated evacuation and admission of nitrogen. Lead tetraacetate (3.58 g, stabilised with ca. 15 % acetic acid) was added and the reaction mixture was again degassed. Warming to from 40 °C to 50 °C initiated the exothermic evolution of carbon dioxide which was complete after 20 min. The solution was cooled and extracted with several portions of pentane. The pentane extracts were washed subsequently with aqueous solutions comprising, in each case, 20 % of perchloric acid, 10 % of aqueous potassium carbonate, and pure deionised water, and dried with sodium sulphate. Finally, the desired product was purified by column chromatography on silica gel eluting with pentane to furnish a yield of 68 % of the chloride 2 (mixture of two isomers) as a clear oil.

### Example 1.2 6-Phenyl-2-propyl-decahydro-azulene (3)

10 ml of benzene and 1.7 g of anhydrous AlCl₃ were mixed thoroughly in a flask placed on a water bath. Chloride 2 of perhydroazulene (3.73 mmol) obtained as described above was added dropwise. Gaseous HCl was evolved and the temperature was increased to about 35 °C. The mixture was heated for four hours to from 45 °C to 50 °C, then it was cooled and poured onto ice; the organic layer was separated and washed with an aqueous solution comprising 5 % HCl and subsequently several times with deionised water. Excess benzene was removed by distillation under reduced pressure, and the product was purified by column chromatography on silica gel eluting with pentane to get 3, in a yield of 62 % as a colourless liquid.

### Example 1.3 4-(2-Alkyl-decahydro-azulen-6-yl)-benzoyl chloride (4)

0.9 g (6.81 mmol) anhydrous aluminium chloride were mixed with 50 ml of dichloromethane. After cooling the solution to -10 °C, a solution of 0.86 g (6.81 mmol) of oxalyl chloride in 10 ml of dichloromethane was added dropwise. The mixture was further stirred at -10 °C for 10 min. The reactant 3 was added to the suspension and stirring was continued for a further five to ten minutes at the same temperature. The reaction mixture was added to 100 g of an ice-water mixture, and extracted with 3 portions of 50 ml each of dichloromethane. The combined organic phase was dried over MgSO₄ and solvent was removed under reduced pressure to get the crude intermediate which was dissolved in 10 ml of chlorobenzene and refluxed for 3 h. The solvent was removed by distillation under reduced pressure to get the crude product as a light yellow liquid which was further purified through column chromatography on silica gel eluting with a mixture of identical volumes of pentane and dichloromethane (1:1) to yield a light yellow liquid in a yield of between 75 % and 80 %.

### Example 1.4 4-(2-Alkyl-decahydro-azulen-6-yl)-benzonitrile (5)

The acid chloride 4 (0.5 mmol) was saturated with dry ammonia for 30 min and the reaction mixture was concentrated to dryness. The residue was washed with water and air dried. After several washings with pentane and pentane/dichloromethane to remove impurities, the intermediate amide was obtained as a colourless solid. In a well-ventilated hood, trichloromethyl chloroformate (0.1 ml) was added drop-wise to a cold (between 0 °C and 5 °C) stirred solution of the amide in trimethyl phosphate (1 ml). The reaction mixture was slowly heated to 60 °C for 10 min to ensure the completion of the reaction and also drive away any generated phosgene. After cooling with an ice-water bath, the reaction mixture was vigorously stirred and ice-water (5 g) was added to destroy any trace of phosgene and chloroformate. The mixture was extracted with three portions of dichloromethane and dried with MgSO₄. The solvent was evaporated in vacuo and the compound was purified by column chromatography on silica gel eluting with pentane and then pentane/dichloromethane mixture to yield a colourless liquid in a yield of 55 % which solidified below 0 °C.

### Example 2

2-propyl-perhydroazylene-6-carboxylic acid was esterified with 4-hydroxybenzonitrile to yield 4-cyanophenyl 2-propyl-6-perhydroazulene carboxylate (compound **A**). White crystals with a crystalline to nematic transition temperature T_{cn} of 80 °C and a nematic to isotropic transition temperature Tₙᵢ of 107 °C were obtained.

In a similar reaction, 2-propyl-perhydroazylene-6-carboxylic acid was esterified with 4-n-butylphenol to yield 4-butylphenyl 2-propyl-6-perhydroazulene carboxylate (compound **B**). White crystals with a crystalline to nematic transition temperature T_{cn} of 35 °C and a nematic to isotropic transition temperature Tₙᵢ of 80 °C were obtained.

The eutectic formed from amount-of-substance fractions of 19 cmol/mol of **A** and 81 cmol/mol of B had a crystalline to nematic transition temperature T_{cn} of 29 °C and a nematic to isotropic transition temperature Tₙᵢ of 97 °C.

### Comparative Example 3

Similar liquid crystal compounds were prepared from 4-n-propyl cyclohexanoic acid and 4-hydroxybenzonitrile (ester **C**, 4-cyanophenyl-4-propylcyclohexanoate), having a crystalline to nematic transition temperature T_{cn} of 55 °C and a nematic to isotropic transition temperature Tₙᵢ of 70 °C, and from 4-n-propyl cyclohexanoic acid and 4-n-amylphenol (ester **D**, 4-n-amylphenyl-4-propylcyclohexanoate), having a crystalline to nematic transition temperature T_{cn} of 32 °C and a nematic) to isotropic transition temperature Tₙᵢ of 38 °C.

A binary eutectic prepared from amount-of-substance fractions of 34 % of **C** and 66 % of **D** had a crystalline to nematic transition temperature T_{cn} of 24 °C and a nematic to isotropic transition temperature Tₙᵢ of 46 °C. It can be seen that for similar chemical structures, the useful liquid crystalline range is markedly broader in the case of a perhydroazulene based structure than for a corresponding cyclohexane derived structure. Moreover, the rotational viscosity of the mixture of example 3 was only 40 % of that of the mixture of comparative example 4. Both findings were unexpected and make surprisingly useful materials.

### Comparative Example 4

Further analogous liquid crystal compounds were prepared from 4-(4-n-propylcyclohexyl) cyclohexanoic acid and 4-hydroxybenzonitrile (ester **E**, 4-cyanophenyl-4-(4-propylcyclohexyl)cyclo-hexanoate), having a crystalline to nematic transition temperature T_{cn} of 93 °C and a nematic to isotropic transition temperature Tₙᵢ of 210 °C, and from 4-(4-n-propylcyclohexyl) cyclohexanoic acid and 4-n-amylphenol (ester **F**, 4-n-amylphenyl-4-(4-propylcyclohexyl)cyclohexanoate), having a crystalline to smectic transition temperature T_{cs} of 34 °C, a smectic to nematic transition temperature Tₛₙ of 157 °C and a nematic to isotropic transition temperature Tₙᵢ of 188 °C.

A binary eutectic prepared from amount-of-substance fractions of 85 % of **E** and 15 % of **F** had a crystalline to nematic transition temperature T_{cn} of 90 °C and a nematic to isotropic transition temperature Tₙᵢ of 207 °C. It can be seen that for a eutectic mixture based on molecules of a similar chemical structure wherein the hydroazulene derived moiety was replaced by a biscyclohexyl derived moiety, the useful liquid crystalline range lies in a temperature region which is not useful for ambient temperature applications.

### Example 5

Ternary eutectic mixtures have been prepared by mixing the compounds described supra as components 1, 2, and 3, in the following amount-of-substance fractions (stated in % = cmol/mol), and the nematic temperature range, A T in K:

**Table 1**

| Mixture | Component 1 | Component 2 | Component 3 | T_{cn} | Tₙᵢ | ΔT/K |
|---|---|---|---|---|---|---|
| 6.1 | **A**, 14 % | **B**, 56 % | **C**, 30 % | 18 °C | 89 °C | 71 |
| 6.2 | **A**, 17 % | **B**, 70 % | **E**, 13 % | 25 °C | 111 °C | 86 |
| 6.3 | **A**,15% | **B**, 40 % | **D**, 45 % | 12 °C | 84 °C | 72 |
| 6.4 | **A**, 14 % | **C**, 28 % | **D**, 58 % | 17 °C | 67 °C | 50 |
| 6.5 | **A**,26% | **C**, 56 % | **E**, 18 % | 35 °C | 135 °C | 100 |
| 6.6 (comp.) | **C**,29% | **D**, 62 % | **E**, 9 % | 20 °C | 63 °C | 43 |

It can be seen that ternary eutectics comprising molecules with perhydroazulene derived moieties exhibit broader nematic temperature ranges.

### Example 6

A four-component eutectic system was prepared by mixing 16 cmol/mol of **A,** 45 cmol/mol of **B,** 13 cmol/mol of **C,** and 26 cmol/mol of **D.** This system had a crystalline to nematic transition temperature T_{cn} of -2 °C and a nematic to isotropic transition temperature Tₙᵢ of 87 °C.

As has been shown, the 2,6-(per)hydroazulenylene diradical is a novel, highly nematogenic building block for molecules exhibiting liquid crystal properties. The values found for rotational viscosity, birefringence, and dielectric anisotropy of liquid crystal materials comprising this novel building block are in the same range as those of analogous liquid crystalline systems based on cyclohexylene and biscyclohexylene moieties. As the molecular dimensions, especially the aspect ratio, of the 2,6-perhydroazulenylene diradical differ from those of the 1,4-cyclohexylene and 4,4'-bis-cyclohexylene diradicals, liquid crystal materials comprising these building blocks have different elastic properties and different characteristic curves for their electrooptical behaviour. Both polar and nonpolar perhydroazulene carboxylic acid esters show a much broader nematic temperature range, compared to analogous molecules based on cyclohexanoic acid esters. See example 2 and comparative example 3, comparing **A** to **C** and **B** to **D. In** contrast to systems based on dialkyl-biscyclohexylene carboxylic acid esters (see comparative example 4) which have broad smectic phases, the perhydroazulene based systems are purely nematic (compare compounds **B** and **E**).

Simple binary and ternary eutectics have room-temperature liquid crystalline phases, and a broad nematic temperature range. See table 1 in Example 5.

## Claims

1. Compounds of formula (i)
R¹ᵢ-A¹ᵢ-(Z²ᵢ-A²ᵢ)ₘ-(Z³ᵢ-A³ᵢ)ₙ-(Z⁴ᵢ-A⁴ᵢ)ₒ-R²ᵢ (i)
wherein at least one of the divalent groups A¹ᵢ, A²ᵢ, A³ᵢ and A⁴ᵢ is a group derived from at least partially hydrogenated azulene, with the bonds in the 2- and 6- positions or in the 6- and 2-positions,
R¹ᵢ and R²_{¡} are each independently selected from the group consisting of hydrogen, an alkyl or alkenyl residue of from 1 to 15 carbon atoms which may optionally be singly substituted with a halogen, -CN or -CF₃ group, wherein in the alkyl or alkenyl residues, one or more methylene -CH₂- groups may be replaced by -O-, -S-, 1,3-cyclobutylene, a keto group -CO-, an ester group -CO-O-, or a carbonate group -O-CO-O-, independently from one another, but in a way that no two oxygen atoms are directly linked to each other,
R²ᵢ may also be selected from the group consisting of -CN, -N=C=S, -S-CN, -F, -Cl, -SF₅, -CF₃, -CH₂F, -CHF₂, -O-CF₃, -O-CF₂-CF₂H, and -O-C₂F₅,
A1ᵢ, A²ᵢ, A³ᵢ and A⁴ᵢ are each independently selected from the group consisting of partially or completely hydrogenated 2,6-azulenylene, partially or completely hydrogenated 6,2-azulenylene, trans-1,4-cyclohexylene, 1,4-cyclohexenylene, 1,4-phenylene, 2-fluoro-1,4-phenylene, 3-fluoro-1,4-phenylene, 5-fluoro-1,4-phenylene, 2,3-, 2,5- and 2,6-difluoro-1,4-phenylene, and 3,5-difluoro-1,4-phenylene, 4,4'-diphenylene, 4,4'-biscyclohexylene, tetrahydropyran-2,5- or 3,6-ylene, 1,3- pyrimidine-2,5-diyl, and 1,3-dioxane-2,5-diyl, with the proviso as mentioned above that at least one of the divalent groups A¹ᵢ, A²ᵢ , A³ᵢ and A⁴ᵢ is a group derived from at least partially hydrogenated azulene, with the bonds to the 2- and 6-, or 6- and 2-positions,
Z²ᵢ, Z³ᵢ and Z⁴ᵢ are independently selected from the group consisting of a single bond, -CH₂-CH₂-, -CF₂-CF₂-, an ester bond -CO-O-, a thioester bond -CO-S-, trans -CH=CH-, -C≡C-, -CH₂-O-and -CF₂-O-, with the proviso that Z^{j}ᵢ may not be an ester or a thioester bond if A^{j-i}ᵢ is a 2,6-(per)hydroazulenylene diradical, or if A^{j}ᵢ is a 6,2-(per)hydroazulenylene diradical,
m, n and o being 0, 1, or 2, independently from each other.

2. Compounds according to claim 1 where
R¹ᵢ is an alkyl residue or an alkoxy residue of from 1 to 15 carbon atoms,
R²ᵢ is selected from the group consisting of -CN, -N=C=S, -F, -Cl, -SF₅, -CF₃, - CHF₂, -CH₂F, -O-CF₃, -O-CHF₂, -O-CH₂F, -O-CF₂-CF₂H, -O-C₂F₅,
A¹ᵢ is dihydro, tetrahydro, hexahydro, octahydro or decahydro azulenylene, preferably tetrahydro hexahydro, octahydro or decahydro azulenylene,
A²ᵢ is 1,4-cyclohexylene or 1,4-phenylene, or if at least one of n and o does not equal zero, tetrahydropyran-2,5- or 3,6-ylene, 1,3-dioxan-2,5-diyl or pyrimidine-2,5-diyl
A³ᵢ 1,4-phenylene, 3-fluoro-1,4-phenylene or 3,5-difluoro-1,4-phenylene
A⁴ᵢ 1,4-phenylene, 3-fluoro-1,4-phenylene, 2,3-difluoro-1,4-phenylene or 3,5-difluoro-1,4-phenylene
Z²ᵢ to Z⁴ᵢ being independently selected from the group consisting of a single bound, an ethane 1,2-diyl group -CH₂-CH₂-, an ethene 1,2-diyl group -CH=CH- and an acetylene group -C_{c}C-.

3. Compounds according to claim 1 where
R¹ᵢ is an alkyl residue or an alkoxy residue of from 1 to 15 carbon atoms,
R²ᵢ is selected from the group consisting of -CN, -N=C=S, -F, -Cl, -SF₅, -CF₃, - CHF₂, CH₂F, -O-CF₃, -O-CHF₂, -O-CH₂F, -O-CF₂-CF₂H, -O-C₂F₅, in each case being cycloaliphatically or aromatically bound,
A⁴ᵢ is dihydro, tetrahydro, hexahydro, octahydro or decahydro azulenylene, preferably tetrahydro hexahydro, octahydro or decahydro azulenylene,
A³ᵢ is 1,4-cyclohexylene or 1,4-phenylene, or if at least one of n and o does not equal zero, 1,3-dioxan-2,5-diyl or pyrimidine-2,5-diyl
A²ᵢ 1,4-phenylene, 3-fluoro-1,4-phenylene or 3,5-difluoro-1,4-phenylene
A¹ᵢ 1,4-phenylene, 3-fluoro-1,4-phenylene, 2,3-difluoro-1,4-phenylene or 3,5-difluoro-1,4-phenylene
Z²ᵢ to Z⁴ᵢ being independently selected from the group consisting of a single bond, an ester group -CO-O-, an ethane-1,2-diyl group -CH₂-CH₂-, an ethene-1,2-diyl group -CH=CH-, and an acetylene group -C≡C- with the proviso that Z^{j}ᵢ may not be an ester bond if A^{j-1}ᵢ is a 2,6-(per)hydroazulenylene diradical, or if A^{j}ᵢ is a 6,2-(per)hydroazulenylene diradical.

4. Compounds of formula (i) according to claim 1
R¹ᵢ-A¹ᵢ-(Z²ᵢ-A²ᵢ)ₘ-(Z³ᵢ-A³ᵢ)ₙ-(Z⁴ᵢ-A⁴ᵢ)ₒ-R²ᵢ (i)
wherein at least one of the divalent groups A¹ᵢ, A²ᵢ, A³ᵢ and A⁴ᵢ is a group derived from at least partially hydrogenated azulene,
R¹ᵢ and R²ᵢ are each independently selected from the group consisting of hydrogen, an alkyl residue of from 1 to 15 carbon atoms which may optionally be singly substituted with a halogen, -CN or -CF₃ group, wherein in the alkyl or alkenyl residues, one or more methylene -CH₂- groups may be replaced by -O-, -S-, 1,3-cyclobutylene, a keto group -CO-, an ester group -CO-O-, or a carbonate group -O-CO-O-, independently from one another, but in a way that no two oxygen atoms are directly linked to each other,
R²ᵢ may also be selected from the group consisting of -CN, -N=C=S, -S-CN, -F, -Cl, -SF₅, -CF₃, -CH₂F, -CHF₂, -O-CF₃, -O-CF₂-CF₂H, and -O-C₂F₅.
A¹ᵢ, A²ᵢ, A³ᵢ and A⁴ᵢ are each independently selected from the group consisting of partially or completely hydrogenated 2,6-azulenylene, 6,2-azulenylene, trans-1,4-cyclohexylene, 1,4-cyclohexenylene, 1,4-phenylene, 2-fluoro-1,4-phenylene, 3-fluoro-1,4-phenylene, 5-fluoro-1,4-phenylene, 2,3-, 2,5- and 2,6-difluoro-1,4-phenylene, and 3,5-difluoro-1,4-phenylene, 4,4'-diphenylene, 4,4'-biscyclohexylene, tetrahydropyran-2,5- or 3,6-ylene, 1,3- pyrimidine-2,5-diyl, and 1,3-dioxane-2,5-diyl, with the proviso as mentioned above that at least one of the divalent groups A¹ᵢ, A²ᵢ, A³ᵢ and A⁴ᵢ is a group derived from at least partially hydrogenated azulene, with the bonds to the 2-and 6-, or 6- and 2-positions, and wherein at least one of the at least partially hydrogenated azulene moieties has at least one fluorine substituent in at least one of the 1, 3, 4, 5, 6, 7, 8, 9, and 10 positions,
Z²ᵢ, Z³ᵢ and Z⁴ᵢ are independently selected from the group consisting of a single bond, an ester bond -CO-O-, a thioester bond -CO-S-, -CH₂-CH₂-, -CF₂-CF₂-, trans - CH=CH-, -C_{c}C-, -CH₂-O-and -CF₂-O-, with the proviso that Z^{j}ᵢ may not be an ester or a thioester bond if A^{j-1}ᵢ is a 2,6-(per)hydroazulenylene diradical, or if A^{j}ᵢ is a 6,2-(per)hydroazulenylene diradical
m, n and o being 0, 1, or 2, independently from each other.

5. A method of use of the compounds according to claim 1 as a constituent of liquid crystalline media for electrooptical display elements which method comprises preparing mixtures that contain at least one of the compounds of claim 1.

6. A mixture exhibiting liquid crystal behaviour comprising a compound according to claim 1.

7. The mixture of claim 6 which comprises more than one compound of claim 1.

8. The mixture of claim 6 exhibiting liquid crystal behaviour comprising at least two compounds of claim 1 according to formula (i)
R¹ᵢ-A¹ᵢ-(Z²ᵢ-A²ᵢ)ₘ-(Z³ᵢ-A³ᵢ)ₙ-(Z⁴ᵢ-A⁴ᵢ)ₒ-R²ᵢ (i)
wherein at least one of the divalent groups A¹ᵢ, A²ᵢ , A³ᵢ and A⁴ᵢ is a group derived from at least partially hydrogenated azulene,
R¹ᵢ and R²ᵢ are each independently selected from the group consisting of hydrogen, an alkyl residue of from 1 to 15 carbon atoms which may optionally be singly substituted with a halogen, -CN or -CF₃ group, wherein in the alkyl or alkenyl residues, one or more methylene -CH₂- groups may be replaced by -O-, -S-, 1,3-cyclobutylene, a keto group -CO-, an ester group -CO-O-, or a carbonate group -O-CO-O-, independently from one another, but in a way that no two oxygen atoms are directly linked to each other,
R²_{¡} may also be selected from the group consisting of -CN, -N=C=S, -S-CN, -F, -Cl, -SF₅, -CF₃, -CH₂F, -CHF₂, -O-CF₃, -O-CF₂-CF₂H, and -O-C₂F_{5.}
A¹ᵢ, A²ᵢ, A³ᵢ and A⁴ᵢ are each independently selected from the group consisting of partially or completely hydrogenated 2,6-azulenylene, 6,2-azulenylene, trans-1,4-cyclohexylene, 1,4-cyclohexenylene, 1,4-phenylene, 2-fluoro-1,4-phenylene, 3-fluoro-1,4-phenylene, 5-fluoro-1,4-phenylene, 2,3-, 2,5- and 2,6-difluoro-1,4-phenylene, and 3,5-difluoro-1,4-phenylene, 4,4'-diphenylene, 4,4'-biscyclohexylene, tetrahydropyran-2,5- or 3,6-ylene, 1,3- pyrimidine-2,5-diyl, and 1,3-dioxane-2,5-diyl, with the proviso as mentioned above that at least one of the divalent groups A¹ᵢ, A²ᵢ, A³ᵢ and A⁴ᵢ is a group derived from at least partially hydrogenated azulene, with the bonds to the 2-and 6-, or 6- and 2-positions.
Z²ᵢ, Z³ᵢ and Z⁴ᵢ are independently selected from the group consisting of a single bond, -CH₂-CH₂-, -CF₂-CF₂-, trans -CH=CH-, -C_{c}C-, -CH₂-O-and -CF₂-O-,
m, n and o being 0, 1, or 2, independently from each other,
wherein the at least two compounds differ in at least one of R¹ᵢ , A¹ᵢ, 2²ᵢ , A²ᵢ, Z³ᵢ, A³ᵢ , Z⁴ᵢ , A⁴ᵢ , R²ᵢ, m, n, and o.

9. The mixture of claim 6 which comprises a mass fraction of from 1 % to 100 %, of one or more compounds of claim 1.

10. The mixture of claim 9 which comprises a mass fraction of
a) from 1 % to 100 % of one or more compounds of the formula (i) of claim 1,
b) from 0 % to 99 % of one or more compounds which form liquid crystalline phases, of the general formula (ii)
R¹ᵢᵢ-A¹ᵢᵢ-Z¹ᵢᵢ-(A²ᵢᵢ-Z²ᵢᵢ)ₙ-A³ᵢᵢ-R²ᵢᵢ (ii)
wherein
A¹ᵢᵢ, A²ᵢᵢ and A³ᵢᵢ are each, independently of one another, selected from the group consisting of trans-1,4-cyclohexylene, 1,4-cyclohexenylene, 1,4-phenylene, 2-fluoro-1,4-phenylene, 3-fluoro-1,4-phenylene, 2,3- and 2,6-difluoro-1,4-phenylene, and 3,5-difluoro-1,4-phenylene, pyrimidine-2,5-diyl, and 1,3-dioxane-2,5-diyl,
-Z¹ᵢᵢ- and -2²ᵢᵢ- represent linking groups selected independently from the group consisting of a single bond, -CH₂-CH₂-, -CF₂-CF₂-, an ester bond -CO-O-, trans - CH=CH-, -C≡C-, and -CF₂-O-,
R²ᵢᵢ stands for -F, -Cl, -O-CF₃, or -CF₂-CF₃, preferably -F, or -CF₃, and
R¹ᵢᵢ is selected from the group consisting of hydrogen, an alkyl residue of from 1 to 15 carbon atoms or an alkylene residue of from 2 to 15 carbon atoms which may optionally be singly substituted with a -CN or -CF₃ group, or substituted with at least one halogen atom, wherein in the alkyl or alkenyl residues, one or more methylene - CH₂- groups may be replaced by -O-, -S-, cyclobutane-1,3-diyl, a keto group -CO-, an ester group -CO-O-, or a carbonate group -O-CO-O-, independently from one another, but in a way that no two oxygen atoms are directly linked to each other,
and
c) from 0 % to 15 % of one or more further liquid crystalline compounds.

11. A liquid crystal display comprising the liquid crystal mixture of one of claims 6 to 10.

12. The mixture of claim 9 which comprises a mass fraction of
a) from 5 % to 50 % of compounds of the formula (i),
b) from 50 % to 95 % of one or more compounds which form liquid crystalline phases, of the general formula (ii)
R¹ᵢᵢ-A¹ᵢᵢ-Z¹ᵢᵢ-(A²ᵢᵢ-Z²ᵢᵢ)ₙ-A³ᵢᵢ-R²ᵢᵢ (ii)
wherein
A¹ᵢᵢ, A²ᵢᵢ and R³ᵢᵢ are each, independently of one another, selected from the group consisting of trans-1,4-cyclohexylene, 1,4-cyclohexenylene, 1,4-phenylene, 2-fluoro-1,4-phenylene, 3-fluoro-1,4-phenylene, 2,3- and 2,6-difluoro-1,4-phenylene, and 3,5-difluoro-1,4-phenylene, pyrimidine-2,5-diyl, and 1,3-dioxane-2,5-diyl,
-Z¹ᵢᵢ- and -Z²ᵢᵢ- represent linking groups selected independently from the group consisting of a single bond, -CH₂-CH₂-, -CF₂-CF₂-, an ester bond -CO-O-, trans - CH=CH-, -C≡C-, and -CF₂-O-,
R²ᵢᵢ stands for -F, -Cl, -O-CF₃, or -CF₂-CF₃, preferably -F, or -CF₃, and
R¹ᵢᵢ is selected from the group consisting of hydrogen, an alkyl residue of from 1 to 15 carbon atoms or an alkylene residue of from 2 to 15 carbon atoms which may optionally be singly substituted with a -CN or -CF₃ group, or substituted with at least one halogen atom, wherein in the alkyl or alkenyl residues, one or more methylene - CH₂- groups may be replaced by -O-, -S-, cyclobutane-1,3-diyl, a keto group -CO-, an ester group -CO-O-, or a carbonate group -O-CO-O-, independently from one another, but in a way that no two oxygen atoms are directly linked to each other,
and
c) from 0 % to 45 % of further liquid crystalline compounds.

## Patentansprüche

1. Verbindungen der Formel (i)
R¹ᵢ-Aⁱᵢ-(Z²ᵢ-A²ᵢ)-(Z³ᵢ-A³ᵢ)-ₙ-(Z⁴ᵢ-A⁴ᵢ)ₒ-R²ᵢ (i)
worin mindestens eine der zweiwertigen Gruppen A¹ᵢ, A²ᵢ, A³ᵢ und A⁴ᵢ für eine von mindestens teilweise hydriertem Azulen abgeleitete Gruppe mit den Bindungen in 2- und 6-Position oder in 6- und 2-Position steht,
R¹ᵢ und R²ᵢ jeweils unabhängig voneinander aus der Gruppe bestehend aus Wasserstoff und einem Alkyl-oder Alkenylrest mit 1 bis 15 Kohlenstoffatomen, der gegebenenfalls einfach durch eine Halogen-, -CN- oder -CF₃-Gruppe substituiert ist, ausgewählt sind, wobei in den Alkyl- oder Alkenylresten eine oder mehrere Methylen-CH₂-Gruppen unabhängig voneinander durch -O-, -S-, 1,3-Cyclobutylen, eine Ketogruppe -CO-, eine Estergruppe -CO-O- oder eine Carbonatgruppe -O-CO-O- ersetzt sein können, aber so, daß keine zwei Sauerstoffatome direkt miteinander verknüpft sind,
R²ᵢ auch aus der Gruppe bestehend aus -CN, -N=C=S, -S-CN, -F, -Cl, -SF₅, -CF₃, -CH₂F, -CHF₂, -O-CF₃, -O-CF₂-CF₂H und -O-C₂F₅ ausgewählt sein kann;
A¹ᵢ, A²ᵢ, A³ᵢ und A⁴ᵢ jeweils unabhängig voneinander aus der Gruppe bestehend aus teilweise oder vollständig hydriertem 2,6-Azulenylen, teilweise oder vollständig hydriertem 6,2-Azulenylen, trans-1,4-Cyclohexylen, 1,4-Cyclohexenylen, 1,4-Phenylen, 2-Fluor-1,4-phenylen, 3-Fluor-1,4-phenylen, 5-Fluor-1,4-phenylen, 2,3-, 2,5- und 2,6-Difluor-1,4-phenylen und 3,5-Difluor-1,4-phenylen, 4,4'-Diphenylen, 4,4'-Biscyclohexylen, Tetrahydropyran-2,5-ylen oder -3,6-ylen, 1,3-Pyrimidin-2,5-diyl und 1,3-Dioxan-2,5-diyl ausgewählt sind, mit der oben erwähnten Maßgabe, daß mindestens eine der zweiwertigen Gruppen A¹ᵢ, A²ᵢ, A³ᵢ und A⁴ᵢ für eine von mindestens teilweise hydriertem Azulen abgeleitete Gruppe mit den Bindungen in 2- und 6-Position oder in 6- und 2-Position steht,
Z²ᵢ, Z³ᵢ und Z⁴ᵢ jeweils unabhängig voneinander aus der Gruppe bestehend aus einer Einfachbindung, -CH₂-CH₂-, -CF₂-CF₂-, einer Esterbindung -CO-O-, einer Thioesterbindung -CO-S-, trans-CH=CH-, -C≡C-, -CH₂-O- und -CF₂-O- ausgewählt sind, mit der Maßgabe, daß Z^{j}ᵢ nicht für eine Ester- oder Thioesterbindung stehen darf, wenn A^{j-1}ᵢ für ein 2,6-(Per)hydroazulenylen-Diradikal steht oder wenn A^{j}ᵢ für ein 6,2-(Per)hydroazulenylen-Diradikal steht,
m, n und o unabhängig voneinander für 0, 1 oder 2 stehen.

2. Verbindungen nach Anspruch 1, wobei
R¹ᵢ für einen Alkylrest oder einen Alkoxyrest mit 1 bis 15 Kohlenstoffatomen steht,
R²ᵢ aus der Gruppe bestehend aus -CN, -N=C=S, -F, -Cl, -SF₅, -CF₃, -CH₂F, -CHF₂, -O-CF₃, -O-CHF₂, -O-CH₂F, -O-CF₂-CF₂H und -O-C₂F₅ ausgewählt ist,
A¹ᵢ für Dihydro-, Tetrahydro-, Hexahydro-, Octahydro- oder Decahydroazulenylen, vorzugsweise Tetrahydro-, Hexahydro-, Octahydro- oder Decahydroazulenylen, steht,
A²ᵢ für 1,4-Cyclohexylen oder 1,4-Phenylen oder dann, wenn n und/oder o nicht gleich null sind, für Tetrahydropyran-2,5-ylen oder -3,6-ylen, 1,3-Dioxan-2,5-diyl oder Pyrimidin-2,5-diyl steht,
A³ᵢ für 1,4-Phenylen, 3-Fluor-1,4-phenylen oder 3,5-Difluor-1,4-phenylen steht,
A⁴ᵢ für 1,4-Phenylen, 3-Fluor-1,4-phenylen, 2,3-Difluor-1,4-phenylen oder 3,5-Difluor-1,4-phenylen steht,
Z²ᵢ, bis Z⁴ᵢ unabhängig voneinander aus der Gruppe bestehend aus einer Einfachbindung, einer Ethan-1,2-diylgruppe -CH₂-CH₂-, einer Ethen-1,2-diyl-gruppe -CH=CH- und einer Acetylengruppe -C≡C- ausgewählt sind.

3. Verbindungen nach Anspruch 1, wobei
R¹ᵢ für einen Alkylrest oder einen Alkoxyrest mit 1 bis 15 Kohlenstoffatomen steht,
R²ᵢ aus der Gruppe bestehend aus -CN, -N=C=S, -F, -Cl, -SF₅, -CF₃, -CHF₂, -CH₂F, -O-CF₃, -O-CHF₂, -O-CH₂F, -O-CF₂-CF₂H und -O-C₂F₅, jeweils cycloaliphatisch oder aromatisch gebunden, ausgewählt ist,
A⁴ᵢ für Dihydro-, Tetrahydro-, Hexahydro-, Octahydro- oder Decahydroazulenylen, vorzugsweise Tetrahydro-, Hexahydro-, Octahydro- oder Decahydroazulenylen, steht,
A³ᵢ für 1,4-Cyclohexylen oder 1,4-Phenylen oder dann, wenn n und/oder o nicht gleich null sind, für 1,3-Dioxan-2,5-diyl oder Pyrimidin-2,5-diyl steht,
A²ᵢ für 1,4-Phenylen, 3-Fluor-1,4-phenylen oder 3,5-Difluor-1,4-phenylen steht,
A¹ᵢ für 1,4-Phenylen, 3-Fluor-1,4-phenylen, 2,3-Difluor-1,4-phenylen oder 3,5-Difluor-1,4-phenylen steht,
Z² bis Z⁴ᵢ, unabhängig voneinander aus der Gruppe bestehend aus einer Einfachbindung, einer Estergruppe -CO-O-, einer Ethan-1,2-diylgruppe -CH₂-CH₂-, einer Ethen-1,2-diylgruppe -CH=CH- und einer Acetylengruppe -C-C- ausgewählt sind, mit der Maßgabe, daß Z^{j}ᵢ nicht für eine Esterbindung stehen darf, wenn A^{j-1}ᵢ für ein 2,6-(Per)hydroazulenylen-Diradikal steht oder wenn A^{j}ᵢ für ein 6,2-(Per)hydroazulenylen-Diradikal steht.

4. Verbindungen der Formel (i) nach Anspruch 1
R¹ᵢ-A¹ᵢ-(Z²ᵢ-A²ᵢ)ₘ-(Z³ᵢ-A³ᵢ)ₙ-(Z⁴ᵢ-A⁴ᵢ)ₒ-R²ᵢ, (i)
worin mindestens eine der zweiwertigen Gruppen A¹ᵢ, A²ᵢ, A³ᵢ und A⁴ᵢ, für eine von mindestens teilweise hydriertem Azulen abgeleitete Gruppe steht,
R¹ᵢ und R²ᵢ jeweils unabhängig voneinander aus der Gruppe bestehend aus Wasserstoff und einem Alkylrest mit 1 bis 15 Kohlenstoffatomen, der gegebenenfalls einfach durch eine Halogen-, -CN-oder -CF₃-Gruppe substituiert ist, ausgewählt sind, wobei in den Alkyl- und Alkenylresten eine oder mehrere Methylen-CH₂-Gruppen unabhängig voneinander durch -O-, -S-, 1,3-Cyclobutylen, eine Ketogruppe -CO-, eine Estergruppe -CO-O- oder eine Carbonatgruppe -O-CO-O- ersetzt sein können, aber so, daß keine zwei Sauerstoffatome direkt miteinander verknüpft sind,
R²ᵢ auch aus der Gruppe bestehend aus -CN, -N=C=S, -S-CN, -F, -Cl, -SF₅, -CF₃, -CH₂F, -CHF₂, -O-CF₃, -O-CF₂-CF₂H und -O-C₂F₅ ausgewählt sein kann;
A¹ᵢ, A²ᵢ, A³ᵢ und A⁴ᵢ jeweils unabhängig voneinander aus der Gruppe bestehend aus teilweise oder vollständig hydriertem 2,6-Azulenylen, 6,2-Azulenylen, trans-1,4-Cyclohexylen, 1,4-Cyclohexenylen, 1,4-Phenylen, 2-Fluor-1,4-phenylen, 3-Fluor-1,4-phenylen, 5-Fluor-1,4-phenylen, 2,3-, 2,5- und 2,6-Difluor-1,4-phenylen und 3,5-Difluor-1,4-phenylen, 4,4'-Diphenylen, 4,4'-Biscyclohexylen, Tetrahydropyran-2,5-ylen oder -3,6-ylen, 1,3-Pyrimidin-2,5-diyl und 1,3-Dioxan-2,5-diyl ausgewählt sind, mit der oben erwähnten Maßgabe, daß mindestens eine der zweiwertigen Gruppen A¹ᵢ, A²ᵢ, A³ᵢ und A⁴ᵢ für eine von mindestens teilweise hydriertem Azulen abgeleitete Gruppe mit den Bindungen in 2- und 6-Position oder in 6- und 2-Position steht, und worin mindestens eine der mindestens teilweise hydrierten Azulengruppierungen mindestens einen Fluorsubstituenten in mindestens einer der 1-, 3-, 4-, 5-, 6-, 7-, 8-, 9- und 10-Positionen aufweist,
Z²ᵢ, Z³ᵢ und Z⁴ᵢ jeweils unabhängig voneinander aus der Gruppe bestehend aus einer Einfachbindung, einer Esterbindung -CO-O-, einer Thioesterbindung -CO-S-, -CH₂-CH₂-, -CF₂-CF₂-, trans-CH=CH-, -C≡C-, -CH₂-O- und -CF₂-O- ausgewählt sind, mit der Maßgabe, daß Z^{j}ᵢ nicht für eine Ester- oder Thioesterbindung stehen darf, wenn A^{j-1}ᵢ, für ein 2,6-(Per)hydroazulenylen-Diradikal steht oder wenn A^{j}ᵢ für ein 6,2-(Per)hydroazulenylen-Diradikal steht, m, n und o unabhängig voneinander für 0, 1 oder 2 stehen.

5. Verfahren zur Verwendung der Verbindungen nach Anspruch 1 als Bestandteil von flüssigkristallinen Medien für elektrooptische Anzeigeelemente, bei dem man Mischungen herstellt, die mindestens eine der Verbindungen nach Anspruch 1 enthalten.

6. Mischung mit Flüssigkristallverhalten, die eine Verbindung nach Anspruch 1 umfaßt.

7. Mischung nach Anspruch 6, die mehr als eine Verbindung nach Anspruch 1 umfaßt.

8. Mischung nach Anspruch 6 mit Flüssigkristallverhalten, die mindestens zwei Verbindungen nach Anspruch 1 der Formel (i)
R¹ᵢ-A¹ᵢ-(Z²ᵢ-A²ᵢ)ₘ-(Z³ᵢ-A³ᵢ)ₙ- (Z⁴ᵢ-A⁴ᵢ)ₒ-R²ᵢ (i)
worin mindestens eine der zweiwertigen Gruppen A¹ᵢ, A²ᵢ, A³ᵢ und A⁴ᵢ für eine von mindestens teilweise hydriertem Azulen abgeleitete Gruppe steht,
R¹ᵢ und R²ᵢ jeweils unabhängig voneinander aus der Gruppe bestehend aus Wasserstoff und einem Alkylrest mit 1 bis 15 Kohlenstoffatomen, der gegebenenfalls einfach durch eine Halogen-, -CN-oder -CF₃-Gruppe substituiert ist, ausgewählt sind, wobei in den Alkyl- und Alkenylresten eine oder mehrere Methylen-CH₂-Gruppen unabhängig voneinander durch -O-, -S-, 1,3-Cyclobutylen, eine Ketogruppe -CO-, eine Estergruppe -CO-O- oder eine Carbonatgruppe -O-CO-O- ersetzt sein können, aber so, daß keine zwei Sauerstoffatome direkt miteinander verknüpft sind,
R²ᵢ auch aus der Gruppe bestehend aus -CN, -N=C=S, -S-CN, -F, -Cl, -SF₅, -CF₃, -CH₂F, -CHF₂, -O-CF₃, -O-CF₂-CF₂H und -O-C₂F₅ ausgewählt sein kann;
A¹ᵢ, A²ᵢ, A³ᵢ, und A⁴ᵢ jeweils unabhängig voneinander aus der Gruppe bestehend aus teilweise oder vollständig hydriertem 2,6-Azulenylen, 6,2-Azulenylen, trans-1,4-Cyclohexylen, 1,4-Cyclohexenylen, 1,4-Phenylen, 2-Fluor-1,4-phenylen, 3-Fluor-1,4-phenylen, 5-Fluor-1,4-phenylen, 2,3-, 2,5- und 2,6-Difluor-1,4-phenylen und 3,5-Difluor-1,4-phenylen, 4,4'-Diphenylen, 4,4'-Biscyclohexylen, Tetrahydropyran-2,5-ylen oder -3,6-ylen, 1,3-Pyrimidin-2,5-diyl und 1,3-Dioxan-2,5-diyl ausgewählt sind, mit der oben erwähnten Maßgabe, daß mindestens eine der zweiwertigen Gruppen A¹ᵢ, A²ᵢ, A³ᵢ und A⁴ᵢ für eine von mindestens teilweise hydriertem Azulen abgeleitete Gruppe mit den Bindungen in 2- und 6-Position oder in 6- und 2-Position steht,
Z²ᵢ, Z³ᵢ und Z⁴ᵢ jeweils unabhängig voneinander aus der Gruppe bestehend aus einer Einfachbindung, -CH₂-CH₂-, -CF₂-CF₂-, trans-CH=CH-, -C≡C-, -CH₂-O-und -CF₂-O- ausgewählt sind,
m, n und o unabhängig voneinander für 0, 1 oder 2 stehen,
umfaßt, wobei sich die mindestens zwei Verbindungen bezüglich R¹ᵢ, A¹ᵢ, Z²ᵢ, A²ᵢ, Z³ᵢ, A³ᵢ, Z⁴ᵢ, A⁴ᵢ, R²ᵢ, m, n und/oder o unterscheiden.

9. Mischung nach Anspruch 6, die einen Massenanteil von 1 % bis 100 % einer oder mehrerer Verbindungen nach Anspruch 1 umfaßt.

10. Mischung nach Anspruch 9, die einen Massenanteil von
a) 1 % bis 100 % einer oder mehrerer Verbindungen der Formel (i) nach Anspruch 1,
b) 0 % bis 99 % einer oder mehrerer flüssigkristalline Phasen bildender Verbindungen der allgemeinen Formel (ii)
R¹ᵢᵢ-A¹ᵢᵢ-Z¹ᵢᵢ-(A²ᵢᵢ-Z²ᵢᵢ)ₙ-A³ᵢᵢ-R²ᵢᵢ (ii)
worin
A¹ᵢᵢ, A²ᵢᵢ und A³ᵢᵢ jeweils unabhängig voneinander aus der Gruppe bestehend aus trans-1,4-Cyclohexylen, 1,4-Cyclohexenylen, 1,4-Phenylen, 2-Fluor-1,4-phenylen, 3-Fluor-1,4-phenylen, 2,3- und 2,6-Difluor-1,4-phenylen, und 3,5-Difluor-1,4-phenylen, Pyrimidin-2,5-diyl und 1,3-Dioxan-2,5-diyl ausgewählt sind,
-Z¹ᵢᵢ- und -Z²ᵢᵢ- für Brückengruppen, die unabhängig aus der Gruppe bestehend aus einer Einfachbindung, -CH₂-CH₂-, -CF₂-CF₂-, einer Esterbindung -CO-O-, trans-CH=CH-, -C≡C- und -CF₂-O- ausgewählt sind, stehen,
R²ᵢᵢ für -F, -Cl , -O-CF₃ oder -CF₂-CF₃, vorzugsweise -F, oder -CF₃ steht und
R¹ᵢᵢ aus der Gruppe bestehend aus Wasserstoff und einem Alkylrest mit 1 bis 15 Kohlenstoffatomen oder einem Alkylenrest mit 2 bis 15 Kohlenstoffatomen, der gegebenenfalls einfach durch eine -CN-oder -CF₃-Gruppe oder durch mindestens ein Halogenatom substituiert sein kann, ausgewählt ist, wobei in den Alkyl- oder Alkenylresten eine oder mehrere Methylen-CH₂-Gruppen unabhängig voneinander durch -O-, -S-, Cyclobutan-1,3-diyl, eine Ketogruppe -CO-, eine Estergruppe -CO-O- oder eine Carbonatgruppe -O-CO-O- ersetzt sein können, aber so, daß keine zwei Sauerstoffatome direkt miteinander verknüpft sind,
und
c) 0 % bis 15 % einer oder mehrerer weiterer flüssigkristalliner Verbindungen
umfaßt.

11. Flüssigkristallanzeige, umfassend die Flüssigkristallmischung nach einem der Ansprüche 6 bis 10.

12. Mischung nach Anspruch 9, die einen Massenanteil von
a) 5 % bis 50 % von Verbindungen der Formel (i),
b) 50 % bis 95 % einer oder mehrerer flüssigkristalline Phasen bildender Verbindungen der allgemeinen Formel (ii)
R¹ᵢᵢ-A¹ᵢᵢ-Z¹ᵢᵢ-(A²ᵢᵢ-Z²ᵢᵢ)-A³ᵢᵢ-R²ᵢᵢ, (ii)
worin
A¹ᵢᵢ, A²ᵢᵢ und A³ᵢᵢ jeweils unabhängig voneinander aus der Gruppe bestehend aus trans-1,4-Cyclohexylen, 1,4-Cyclohexenylen, 1,4-Phenylen, 2-Fluor-1,4-phenylen, 3-Fluor-1,4-phenylen, 2,3- und 2,6-Difluor-1,4-phenylen und 3,5-Difluor-1,4-phenylen, Pyrimidin-2,5-diyl und 1,3-Dioxan-2,5-diyl ausgewählt sind,
-Z¹ᵢᵢ- und -Z²ᵢᵢ- für Brückengruppen, die unabhängig aus der Gruppe bestehend aus einer Einfachbindung, -CH₂-CH₂- , -CF₂-CF₂-, einer Esterbindung -CO-O-, trans-CH=CH-, -C≡C- und -CF₂-O- ausgewählt sind, stehen,
R²ᵢᵢ für -F, -Cl, -O-CF₃ oder -CF₂-CF₃, vorzugsweise -F, oder -CF₃ steht und
R¹ᵢᵢ aus der Gruppe bestehend aus Wasserstoff und einem Alkylrest mit 1 bis 15 Kohlenstoffatomen oder einem Alkylenrest mit 2 bis 15 Kohlenstoffatomen, der gegebenenfalls einfach durch eine -CN-oder -CF₃-Gruppe oder durch mindestens ein Halogenatom substituiert sein kann, ausgewählt ist, wobei in den Alkyl- oder Alkenylresten eine oder mehrere Methylen-CH₂-Gruppen unabhängig voneinander durch -O-, -S-, Cyclobutan-1,3-diyl, eine Ketogruppe -CO-, eine Estergruppe -CO-O- oder eine Carbonatgruppe -O-CO-O- ersetzt sein können, aber so, daß keine zwei Sauerstoffatome direkt miteinander verknüpft sind,
und
c) 0 % bis 45 % weiterer flüssigkristalliner Verbindungen
umfaßt.

## Revendications

1. Composés de formule (i)
R¹_{¡}-A¹ᵢ(Z²ᵢ-A²_{¡})ₘ-(Z³ᵢ-A³ᵢ)ₙ-(Z⁴ᵢ-A⁴ᵢ)ₒ-R²ᵢ (i)
dans laquelle au moins un des groupes divalents A¹ᵢ, A²ᵢ, A³ᵢ et A⁴ᵢ est un groupe dérivé d'au moins un azulène partiellement hydrogéné, avec les liaisons dans les positions 2 et 6 ou dans les positions 6 et 2,
R¹ᵢ et R²ᵢ sont chacun indépendamment choisis dans le groupe comprenant un atome d'hydrogène, un résidu d'alkyle ou d'alcényle de 1 à 15 atomes de carbone qui peut être éventuellement substitué de manière unique par un atome d'halogène, un groupe -CN ou -CF₃, dans laquelle dans les résidus d'alkyle ou d'alcényle, un ou plusieurs groupes de méthylène-CH₂- peuvent être remplacés par -O-, -S-, 1,3-cyclobutylène, un groupe cétone -CO-, un groupe ester -CO-O-, ou un groupe carbonate -O-CO-O-, indépendamment les uns des autres, mais de manière à ce que deux atomes d'oxygène ne soient pas directement liés l'un à l'autre,
R²ᵢ peut également être choisi dans le groupe comprenant -CN, -N=C=S, -S-CN, -F, -Cl, -SF₅, - CF₃, -CH₂F, -CHF₂, -O-CF₃, -O-CF₂-CF₂H et -O-C₂F₅,
A¹ᵢ, A²ᵢ, A³ᵢ et A⁴ᵢ sont chacun indépendamment choisis dans le groupe comprenant 2,6-azulénylène partiellement ou complètement hydrogéné, 6,2-azulénylène partiellement ou complètement hydrogéné, trans-1,4-cyclohexylène, 1,4-cyclohexénylène, 1,4-phénylène, 2-fluoro-1,4-phénylène, 3-fluoro-1,4-phénylène, 5-fluoro-1,4-phénylène, 2,3-, 2,5- et 2,6-difluoro-1,4-phénylène et 3,5-difluoro-1,4-phénylène, 4,4'-diphénylène, 4,4'-biscyclohexylène, tétrahydropyran-2,5- ou 3,6-ylène, 1,3- pyrimidine-2,5-diyle et 1,3-dioxane-2,5-diyle à condition, comme susmentionné, qu'au moins un des groupes divalents A¹ᵢ, A²ᵢ, A³ᵢ et A⁴ᵢ soit un groupe dérivé d'au moins l'azulène partiellement hydrogéné, avec les liaisons aux positions 2 et 6 ou 6 et 2,
Z²ᵢ, Z³ᵢ et Z⁴ᵢ sont chacun indépendamment choisis dans le groupe comprenant une liaison simple, -CH₂-CH₂-, -CF₂-CF₂-, une liaison d'ester -CO-O-, une liaison de thioester -CO-S-, trans -CH=CH-, -C=C-, -CH₂-O-et -CF₂-O-, à condition que Z^{j}ᵢ puisse ne pas être une liaison d'ester ou de thioester si A^{j-1}ᵢ est un biradical de 2,6-(per)hydro-azulénylène ou si A^{j}ᵢ est un biradical de 6,2-(per)hydroazulénylène,
m, n et o étant 0, 1 ou 2 indépendamment l'un de l'autre.

2. Composés selon la revendication 1, où
R¹ᵢ est un résidu d'alkyle ou un résidu d'alcoxy de 1 à 15 atomes de carbone,
R²ᵢ est choisi dans le groupe comprenant -CN, - N=C=S, -F, -Cl, -SF₅, -CF₃, -CHF₂, -CH₂F, -O-CF₃, -O-CHF₂, -O-CH₂F, -O-CF₂-CF₂H, -O-C₂F₅,
A¹ᵢ est dihydro, tétrahydro, hexahydro, octahydro ou décahydro azulénylène, de préférence tétrahydro, hexahydro, octahydro ou décahydro azulénylène,
A²ᵢ est 1,4-cyclohéxylène ou 1,4-phénylène ou si au moins l'un de n et o n'est pas égal à zéro, tétrahydropyran-2,5- ou 3,6-ylène, 1,3-dioxan-2,5-diyle ou pyrimidine-2,5-diyle
A³ᵢ 1,4-phénylène, 3-fluoro-1,4-phénylène ou 3,5-difluoro-1,4-phénylène
A⁴ᵢ 1,4-phénylène, 3-fluoro-1,4-phénylène, 2,3-difluoro-1,4-phénylène ou 3,5-difluoro-1,4-phénylène
Z²ᵢ à Z⁴ᵢ étant chacun indépendamment choisi dans le groupe comprenant une liaison simple, un groupe éthane 1,2-diyle -CH₂-CH₂-, un groupe éthène 1,2-diyle -CH=CH- et un groupe acétylène -C_{c}C-.

3. Composés selon la revendication 1, où
R¹ᵢ est un résidu d'alkyle ou un résidu d'alcoxy de 1 à 15 atomes de carbone,
R²ᵢ est choisi dans le groupe comprenant -CN, - N=C=S, -F, -Cl, -SF₅, -CF₃, -CHF₂, CH₂F, -O-CF₃, -O-CHF₂, -O-CH₂F, -O-CF₂-CF₂H, -O-C₂F₅, étant dans chaque cas lié cycloaliphatiquement ou aromatiquement,
A⁴ᵢ est dihydro, tétrahydro, hexahydro, octahydro ou décahydro azulénylène, de préférence, tétrahydro, hexahydro, octahydro ou décahydro azulénylène,
A³ᵢ est 1,4-cyclohexylène ou 1,4-phénylène ou si au moins l'un de n et o n'est pas égal à zéro, 1,3-dioxan-2,5-diyle ou pyrimidine-2,5-diyle
A²ᵢ 1,4-phénylène, 3-fluoro-1,4-phénylène ou 3,5-difluoro-1,4-phénylène
A¹ᵢ 1,4-phénylène, 3-fluoro-1,4-phénylène, 2,3-difluoro-1,4-phénylène ou 3,5-difluoro-1,4-phénylène
Z²ᵢ à Z⁴ᵢ étant chacun indépendamment choisi dans le groupe comprenant une liaison simple, un groupe ester -CO-O-, un groupe éthane-1,2-diyle -CH₂-CH₂-, un groupe éthène-1,2-diyle -CH=CH- et un groupe acétylène -C≡C- à la condition que Z^{j}ᵢ puisse ne pas être une liaison d'ester si A^{j-1}ᵢ est un biradical de 2,6-(per)hydro-azulénylène ou si A^{j}ᵢ est un biradical de 6,2-(per)hydroazulénylène.

4. Composés de formule (i) selon la revendication 1
R¹ᵢ-A¹ᵢ-(Z²ᵢ-A²ᵢ)ₘ-(Z³ᵢ-A³ᵢ)ₙ-(Z⁴ᵢ- A⁴ᵢ)ₒ-R²ᵢ (i)
dans laquelle au moins l'un des groupes divalents A¹ᵢ, A²ᵢ, A³ᵢ et A⁴ᵢ est un groupe dérivé d'au moins l'azulène partiellement hydrogéné,
R¹ᵢ et R²ᵢ sont chacun indépendamment choisis dans le groupe comprenant de un atome d'hydrogène, un résidu d'alkyle de 1 à 15 atomes de carbone qui peuvent être éventuellement substitués de manière unique par un halogène, un groupe -CN ou -CF₃, dans laquelle dans les résidus d'alkyle ou d'alcényle, un ou plusieurs groupes de méthylène -CH₂- peuvent être remplacés par -O-, -S-, 1,3-cyclobutylène, un groupe cétone -CO-, un groupe ester -CO-O-, ou un groupe carbonate -O-CO-O-, indépendamment les uns des autres, mais de manière à ce que deux atomes d'oxygène ne soient pas directement liés l'un à l'autre,
R²ᵢ peut également être choisi dans le groupe comprenant -CN, -N=C=S, -S-CN, -F, -Cl, -SF₅, - CF₃, -CH₂F, -CHF₂, -O-CF₃, -O-CF₂-CF₂H et -O-C₂F₅,
A¹ᵢ, A²ᵢ, A³ᵢ et A⁴ᵢ sont chacun indépendamment choisis dans le groupe comprenant 2,6-azulénylène partiellement ou complètement hydrogéné, 6,2-azulénylène, trans-1,4-cyclohexylène, 1,4-cyclohexénylène, 1,4-phénylène, 2-fluoro-1,4-phénylène, 3-fluoro-1,4-phénylène, 5-fluoro-1,4-phénylène, 2,3-, 2,5- et 2,6-difluoro-1,4-phénylène et 3,5-difluoro-1,4-phénylène, 4,4'-diphénylène, 4,4'-biscyclohexylène, tétrahydropyran-2,5- ou 3, 6-ylène, 1,3-pyrimidine-2,5-diyle et 1,3-dioxane-2,5-diyle, à condition, comme susmentionné, qu'au moins un des groupes divalents A¹ᵢ, A²ᵢ, A³ᵢ et A⁴ᵢ soit un groupe dérivé d'au moins l'azulène partiellement hydrogéné, avec les liaisons aux positions 2 et 6 ou 6 et 2, et dans laquelle au moins un des groupes caractéristiques d'azulène partiellement hydrogéné possède au moins un substituant de fluor dans au moins une des positions 1, 3, 4, 5, 6, 7, 8, 9 et 10,
Z²ᵢ, Z³ᵢ et Z⁴ᵢ sont chacun indépendamment choisis dans le groupe comprenant une liaison simple, une liaison d'ester -CO-O-, une liaison de thioester -CO-S-, -CH₂-CH₂-, -CF₂-CF₂-, trans-CH=CH=, -C_{c}C-, -CH₂-O- et - CF₂-O-, à la condition que Z^{j}ᵢ puisse ne pas être une liaison d'ester ou de thioester si A^{j-1}ᵢ est un biradical de 2,6-(per)hydroazulénylène ou si A^{j}ᵢ est un biradical de 6,2-(per)hydroazulénylène,
m, n et o étant 0, 1 ou 2 indépendamment l'un de l'autre.

5. Procédé d'utilisation des composés selon la revendication 1 en tant que constituant de milieu cristallin liquide pour éléments d'affichage électro-optiques, lequel procédé consiste à préparer des mélanges qui contiennent au moins l'un des composés de la revendication 1.

6. Mélange présentant un comportement de cristal liquide comprenant un composé selon la revendication 1.

7. Mélange selon la revendication 6 comprenant plusieurs composés de la revendication 1.

8. Mélange selon la revendication 6 présentant un comportement du cristal liquide comprenant au moins deux composés de la revendication 1 selon la formule (i)
R¹ᵢ-A¹ᵢ-(Z²ᵢ-A²ᵢ)ₘ-(Z³ᵢ-A³ᵢ)ₙ-(Z⁴ᵢ-A⁴ᵢ)ₒ-R²ᵢ (i)
dans laquelle au moins l'un des groupes divalents A¹ᵢ, A²ᵢ, A³ᵢ et A⁴ᵢ est un groupe dérivé d'au moins un azulène partiellement hydrogéné,
R¹ᵢ et R²ᵢ sont chacun indépendamment choisis dans le groupe comprenant un atome d'hydrogène, un résidu d'alkyle de 1 à 15 atomes de carbone qui peuvent être éventuellement substitués de manière unique par un atome d'halogène, un groupe -CN ou -CF₃, dans laquelle dans les résidus d'alkyle ou d'alcényle, un ou plusieurs groupes de méthylène-CH₂ peuvent être remplacés par -O-, -S-, 1,3-cyclobutylène, un groupe cétone -CO-, un groupe ester -CO-O-, ou un groupe carbonate -O-CO-O-, indépendamment les uns des autres, mais de manière à ce que deux atomes d'oxygène ne soient pas directement liés l'un à l'autre,
R²ᵢ peut également être choisi dans le groupe comprenant -CN, -N=C=S, -S-CN, -F, -Cl, -SF₅, -CF₃, - CH₂F, -CHF₂, -O-CF₃, -O-CF₂-CF₂H et -O-C₂F₅,
A¹ᵢ, A²ᵢ, A³ᵢ et A⁴ᵢ sont chacun indépendamment choisis dans le groupe comprenant 2,6-azulénylène partiellement ou complètement hydrogéné, 6,2-azulénylène, trans-1,4-cyclohexylène, 1,4-cyclohexénylène, 1,4-phénylène, 2-fluoro-1,4-phénylène, 3-fluoro-1,4-phénylène, 5-fluoro-1,4-phénylène, 2,3-, 2,5- et 2,6-difluoro-1,4-phénylène et 3,5-difluoro-1,4-phénylène, 4,4'-diphénylène, 4,4'-biscyclohexylène, tétrahydropyran-2,5- ou 3, 6-ylène, 1,3-pyrimidine-2,5-diyle et 1,3-dioxane-2,5-diyle, à condition, comme susmentionné, qu'au moins un des groupes divalents A¹ᵢ, A²ᵢ, A³ᵢ et A⁴ᵢ, soit un groupe dérivé d'au moins l'azulène partiellement hydrogéné, avec les liaisons aux positions 2 et 6 ou 6 et 2.
Z²ᵢ, Z³ᵢ et Z⁴ᵢ sont chacun indépendamment choisis dans le groupe comprenant une liaison simple, -CH₂-CH₂-, -CF₂-CF₂-, trans -CH=CH-, -C_{c}C-, -CH₂-O- et -CF₂-O-,
m, n et o étant 0, 1 ou 2 indépendamment l'un de l'autre,
dans laquelle les au moins deux composés diffèrent dans au moins l'un de R¹ᵢ, A¹ᵢ, Z²ᵢ, A²ᵢ, Z³ᵢ , A³ᵢ, Z⁴ᵢ, A⁴ᵢ, R²ᵢ, m, n et 0 .

9. Mélange selon la revendication 6 qui comprend une fraction de masse de 1 % à 100 % d'un ou plusieurs composés de la revendication 1.

10. Mélange selon la revendication 9 qui comprend une fraction de masse
a) de 1% à 100 % d'un ou plusieurs composés de la formule (i) de la revendication 1,
b) de 0 % à 99 % d'un ou plusieurs composés qui forment les phases cristallines liquides de la formule générale (ii)
R¹ᵢᵢ-A¹ᵢᵢ-Z¹ᵢᵢ-(A²ᵢᵢ-Z²ᵢᵢ)ₙ-A³ᵢᵢ-R²ᵢᵢ (ii)
dans laquelle
A¹ᵢᵢ, A²ᵢᵢ et A³ᵢᵢ sont chacun, indépendamment les uns des autres choisis dans le groupe comprenant trans-1,4-cyclohexylène, 1,4-cyclohexénylène, 1,4-phénylène, 2-fluoro-1,4-phénylène, 3-fluoro-1,4-phénylène, 2,3- et 2,6-difluoro-1,4-phénylène et 3,5-difluoro-1,4-phénylène, pyrimidine-2,5-diyle et 1,3-dioxane-2,5-diyle,
-Z¹ᵢᵢ et -Z²ᵢᵢ représentent des groupes de liaison choisis indépendamment dans le groupe comprenant une liaison simple, -CH₂-CH₂-, -CF₂-CF₂-, une liaison ester -CO-O-, trans -CH=CH-, -C ≡ C- et -CF₂-O-,
R²ᵢᵢ signifie -F, -Cl, -O-CF₃ ou -CF₂-CF₃-, de préférence, -F ou -CF₃, et
R¹ᵢᵢ est choisi dans le groupe comprenant un atome d'hydrogène, un résidu d'alkyle de 1 à 15 atomes de carbone ou un résidu d'alkylène de 2 à 15 atomes de carbone qui peuvent être éventuellement substitués de manière unique par un groupe -CN ou -CF₃, ou substitué par au moins un atome d'halogène, dans laquelle dans les résidus d'alkyle ou d'alcényle, un ou plusieurs groupes de méthylène-CH₂ peuvent être remplacés par -O-, -S-, cyclobutane-1,3-diyle, un groupe cétone -CO-, un groupe ester -CO-O- ou un groupe carbonate -O-CO-O-, indépendamment les uns des autres, mais de manière à ce que deux atomes d'oxygène ne soient pas directement liés l'un à l'autre,
et
c) de 0 % à 15 % d'un ou plusieurs autres composés cristallins liquides.

11. Affichage à cristaux liquides comprenant le mélange de cristaux liquides d'une des revendications 6 à 10.

12. Mélange selon la revendication 9, comprenant une fraction de masse de
a) de 5 % à 50 % des composés de la formule (i),
b) de 50 % à 95 % d'un ou plusieurs composés qui forment les phases cristallines liquides, de la formule générale (ii)
R¹ᵢᵢ-A¹ᵢᵢ-Z¹ᵢᵢ-(A²ᵢᵢ-Z²ᵢᵢ)ₙ-A³_{¡¡}-R²_{¡¡} (ii)
dans laquelle
A¹ᵢᵢ, A²ᵢᵢ et A³ᵢᵢ sont chacun, indépendamment les uns des autres choisis dans le groupe comprenant trans-1,4-cyclohexylène, 1,4-cyclohexénylène, 1,4-phénylène, 2-fluoro-1,4-phénylène, 3-fluoro-1,4-phénylène, 2,3- et 2,6-difluoro-1,4-phénylène et 3,5-difluoro-1,4-phénylène, pyrimidine-2,5-diyle et 1,3-dioxane-2,5-diyle,
-Z¹ᵢᵢ et -Z²ᵢᵢ représentent des groupes de liaison choisis indépendamment dans le groupe comprenant une liaison simple, -CH₂-CH₂-, -CF₂-CF₂-, une liaison d'ester -CO-O-, trans -CH=CH-, -C ≡ C- et -CF₂-O-,
R²ᵢᵢ représente -F, -Cl, -O-CF₃ ou -CF₂-CF₃-, de préférence, -F ou -CF₃, et
R¹ᵢᵢ est choisi dans le groupe comprenant un atome d'hydrogène, un résidu d'alkyle de 1 à 15 atomes de carbone ou un résidu d'alkylène de 2 à 15 atomes de carbone qui peuvent être éventuellement substitués de manière unique par un groupe -CN ou -CF₃, ou substitué par au moins un atome d'halogène, dans laquelle dans les résidus d'alkyle ou d'alcényle, un ou plusieurs groupes de méthylène-CH₂ peuvent être remplacés par -O-, -S-, cyclobutane-1,3-diyle, un groupe cétone -CO-, un groupe ester -CO-O- ou un groupe carbonate -O-CO-O-, indépendamment les uns des autres, mais de manière à ce que deux atomes d'oxygène ne soient pas directement liés l'un à l'autre,
et
c) de 0 % à 45 % d'autres composés cristallins liquides.
